(19) Europäisches Patentamt
European Patent Office
Office européen des brevets

(11) **EP 2 515 814 B1**

(12) **EUROPEAN PATENT SPECIFICATION**

(45) Date of publication and mention of the grant of the patent:
**14.03.2018 Bulletin 2018/11**

(51) Int Cl.:
*A61F 13/15* (2006.01)　　*B32B 27/02* (2006.01)
*D02G 3/02* (2006.01)　　*B32B 37/06* (2006.01)
*B32B 37/20* (2006.01)

(21) Application number: **10843488.7**

(22) Date of filing: **15.12.2010**

(86) International application number:
**PCT/US2010/060487**

(87) International publication number:
**WO 2011/087692 (21.07.2011 Gazette 2011/29)**

(54) **STRETCH ARTICLES INCLUDING POLYOLEFIN ELASTIC FIBER**

STRECKARTIKEL MIT ELASTISCHEN POLYOLEFINFASERN

ARTICLES ÉLASTIQUES COMPRENANT UNE FIBRE ÉLASTIQUE DE POLYOLÉFINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.12.2009 US 289818 P**

(43) Date of publication of application:
**31.10.2012 Bulletin 2012/44**

(73) Proprietor: **Invista Technologies S.à.r.l.**
**9000 St. Gallen (CH)**

(72) Inventors:
• **BING-WO, Ronald, D.**
**Waynesboro**
**VA 22980 (US)**

• **NGUYEN, Young, D.**
**Crozet**
**VA 22932 (US)**
• **LAMBERT, James, Michael**
**Staunton**
**VA 22401 (US)**

(74) Representative: **Cockerton, Bruce Roger**
**Carpmaels & Ransford LLP**
**One Southampton Row**
**London WC1B 5HA (GB)**

(56) References cited:
**WO-A2-2006/017518**　　**US-A- 5 843 068**
**US-A1- 2004 005 834**　　**US-A1- 2006 148 358**
**US-A1- 2007 044 905**　　**US-A1- 2007 141 354**
**US-A1- 2008 132 872**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE DISCLOSURE

This disclosure relates to stretch articles including stretch yarns of elastomeric propylene-based polymer fibers capable of being bound directly to a substrate without adhesives and to methods of making the stretch articles.

BACKGROUND

Materials with excellent stretchability and elasticity are needed to manufacture a variety of disposal and durable stretch articles such as, for example, incontinence pads, disposable diapers, training pants, sports apparel, general apparel and furniture upholstery.

Disposable articles are typically elastic composite materials prepared from a combination of polymer film, fibers, sheets and absorbent materials as well as a combination of fabrication technologies. Whereas the fibers are prepared by well known extrusion/spinning processes such as spun bonding, melt blowing, melt spinning and continuous filament winding techniques, the film and sheet forming processes typically involve known bulk extrusion and coextrusion techniques, e.g., blown film, cast film, profile extrusion, injection molding, extrusion coating, and extrusion sheeting.

Various elastic composites are known for use in connection with stretch articles including, but not limited to, absorbent articles such as infant diapers, training pants, adult incontinence products and the like. Such elastic composites are generally employed along the longitudinal side edges or lateral end edges of an absorbent article such as a diaper. The elastic composites are generally thought to be particularly well suited for improving the fit and containment of the absorbent article. Elastic portions are typically constructed into diaper waist band portions to prevent the diaper from failing (such as shown in U.S. Pat. No. 4,381,781 (Sciaraffa)) and leg cuff portions to prevent leakage. Often, the elastic portions promote better form fitting and/or fastening systems for a good combination of comfort and reliability.

Many constructions of elastic composites or stretch articles are known. Generally, an elastic material, such as a stretch yarn, is contained between two opposing webs. The webs are joined together forming a tube or channel containing the elastic material. Further, the elastic material is attached in some manner to one or both of the opposing webs such that the elastic material gathers the elastic composite.

In order to allow the elastic material to gather the elastic composite, the elastic material is generally adhesively attached to the elastic composite. Adhesive attachment of the elastic material to the elastic composite results in a certain amount of the elastic nature of the elastic material being destroyed. Specifically, at the points where the elastic material is adhesively attached to the elastic composite, the elastic material is no longer capable of being stretched. That is, it is no longer "elastic". Moreover, the use of adhesives to attach the elastic material, or in joining the two layers of material, can be expensive, time-consuming, messy, and has been found, in some instances, to undesirably affect the stiffness of the elastic composite.

The elastomeric materials, or stretch yarns typically employed to make the stretch articles, such as those described above, are made with either spandex or rubber threads. These materials typically require a relatively expensive gluing process and expensive elastic adhesive in the construction. In addition, since spandex and rubber yarns typically do not bond readily to the substrate materials, a relatively large amount of glue/adhesive must be used and care must be taken during gluing process to reduce the amount of creep (separation between the stretch threads and the substrate materials) that occurs during use of the article. Elastic attachment adhesive having a high melting point and quick set are typically used to improve bonding between the yarn and the substrate in order to reduce creep. Several different types of gluing applications are utilized by the industry, each having its own advantages relative to cost and creep performance.

Several ultrasonic techniques have been proposed for use to bond the layers of the article together in such a way as to secure (e.g., via entrapment) the elastic materials to the opposing layers of the article. One such technique utilizes a solid bar of material that bonds completely over the elastic material as well as the opposing web on both sides of the elastic material. A second technique elongates the elastic material, causing it to narrow, and then bonds the opposing webs together forming a tube slightly larger than the narrowed elastic. When the elastic material relaxes, it swells and becomes "trapped" in the tube. A third technique utilizes an elastic "band" which is relatively wide where the opposing webs are bonded together through the elastic "band".

Each of the above techniques present drawbacks in either the processability of the composite, the type of elastic material the can be utilized, or the attributes of the finished elastic composite. None of these techniques involve the bonding of the elastomeric material to the substrate material of the article, thus the elastomeric or stretch yarn is still able to move somewhat with respect to the adjacent layers of substrate material, increasing the risk of creep. As a result, there has remained a need to provide an improved method for bonding stretch yarns to substrate materials of stretch articles, such as the disposable personal care articles described above.

The prior art D1 (US-A-2007/044905) discloses an apparatus for making an elastic laminate including a crosslinked elastic layer formed of an elastic copolymer. The apparatus disclosed by D1 includes a die for extruding a layer of crosslinkable elastic copolymer, at least one roll for conveying the elastic layer, a supply source for providing at least one nonwoven web, a lamination nip for laminating at least one nonwoven web to the elastic layer, a processing unit for crosslinking the elastic layer and a winder for collecting the elastic laminate. The crosslinkable elastic copolymer may be a crosslinkable elastic styrenic block copolymer, a crosslinkable semi-crystalline polyolefin plastomer, or a crosslinkable propylene-ethylene copolymer. The crosslinkable elastic layer may be extruded in the form of a plurality of parallel filaments such as by a filament die. The processing unit may be positioned at several points in the apparatus and may be an electron beam processing unit.

SUMMARY

Briefly described, embodiments of this disclosure include, among others, a stretch article including an elastomeric propylene-based polymer stretch yarn bonded to a substrate material, articles of manufacture including the stretch article (such a as, but not limited to, laminates, fabrics, garments, and textiles including the fiber), and methods of making the stretch article.

One exemplary stretch article according to the present disclosure includes a substrate and at least one stretch yarn made of an elastomeric propylene-based polymer, where the stretch yarn is bonded to the substrate and is capable of being mechanically bonded directly to the substrate surface. In an exemplary embodiment the stretch article including the stretch yarn is a wearable disposable article selected from the group including, but not limited to, diapers, incontinence articles, and personal hygiene articles. In an exemplary embodiment the stretch-yarn has an elongated and/or flat cross-sectional shape.

One exemplary process for preparing an elastomeric propylene-based polymer fiber, among others, includes: providing a substrate material; providing a stretch yarn comprising an elastomeric propylene-based polymer; and bonding the stretch yarn to the substrate material such that at least a portion of the stretch yarn is mechanically bonded directly to the substrate material.

BRIEF DESCRIPTION OF THE DRAWINGS

Many aspects of this disclosure can be better understood with reference to the following drawings. The components in the drawings are not necessarily to scale.

FIG. 1 illustrates an exemplary stretch article of the present disclosure as a diaper showing the location of the stretch yarn of the present disclosure in the leg cuffs and belly bands of the diaper.

FIG. 2 illustrates an exemplary system used to carry out the method of making the stretch articles of the present disclosure and bonding the stretch yarn between layers of an exemplary stretch article of the present disclosure.

DETAILED DESCRIPTION

Before the present disclosure is described in greater detail, it is to be understood that this disclosure is not limited to particular embodiments described, as such may, of course, vary. It is also to be understood that the terminology used herein is for the purpose of describing particular embodiments only, and is not intended to be limiting, since the scope of the present disclosure will be limited only by the appended claims.

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present disclosure, the preferred methods and materials are now described.

As will be apparent to those of skill in the art upon reading this disclosure, each of the individual embodiments described and illustrated herein has discrete components and features that may be readily separated from or combined with the features of any of the other several embodiments without departing from the scope or spirit of the present disclosure. Any recited method can be carried out in the order of events recited or in any other order that is logically possible.

Embodiments of the present disclosure will employ, unless otherwise indicated, techniques of chemistry, fiber technology, textiles, and the like, which are within the skill of the art. Such techniques are fully explained in the literature.

The following examples are put forth so as to provide those of ordinary skill in the art with a complete disclosure and description of how to perform the methods and use the compositions and compounds disclosed and claimed herein. Efforts have been made to ensure accuracy with respect to numbers (*e.g.,* amounts, temperature, *etc.*), but some errors and deviations should be accounted for. Unless indicated otherwise, parts are parts by weight, temperature is in °C, and pressure is in atmospheres. Standard temperature and pressure are defined as 25 °C and 1 atmosphere.

Before the embodiments of the present disclosure are described in detail, it is to be understood that, unless otherwise indicated, the present disclosure is not limited to particular materials, reagents, reaction materials, manufacturing processes, or the like, as such can vary. It is also to be understood that the terminology used herein is for purposes of describing particular embodiments only, and is not intended to be limiting. It is also possible in the present disclosure that steps can be executed in different sequence where this is logically possible.

It must be noted that, as used in the specification and the appended claims, the singular forms "a," "an," and "the" include plural referents unless the context clearly dictates otherwise. Thus, for example, reference to "a support" includes a plurality of supports. In this specification and in the claims that follow, reference will be made to a number of terms that shall be defined to have the following meanings unless a contrary intention is apparent.

Definitions

As used herein, the term "stretch article" refers to an article including an elastic portion and/or the elastic portion of an article including the elastic portion. For instance, a diaper includes a stretch article (e.g., an elastomeric composite) to form the leg cuffs and/or belly band of the diaper; however, in some instances one may refer to the diaper itself as a stretch article. As used herein, as stretch article as used herein refers to any generally elastomeric article of manufacture including the bonded stretch yarn described herein.

As used herein, the term "fiber" refers to filamentous material that can be used in fabric and yarn as well as textile fabrication. One or more fibers can be used to produce a fabric or yarn. The yarn can be fully drawn or textured according to methods known in the art. As used herein the term "stretch yarn" refers to one or more elastomeric fibers used in the manufacture of elastic portions of the stretch articles described in the present disclosure. In embodiments, the stretch yarn is made from an elastomeric propylene-based polymer fiber.

As used herein, the term "substrate material" refers to a material used in the manufacture of a stretch article, as described herein, to which a stretch yarn is bonded. The substrate material may include one or more layers of material, in which the layers may be of the same or different material. Typically, the substrate material is a layer of fabric or nonwoven used in the manufacture of a stretch article such as a diaper or other disposable absorbent article.

As used herein, the term "substantially free of", in reference to a chemical, indicates that the chemical is intended to be excluded from an object (e.g., a fiber); however, trace amounts of the chemical might be present in/on the object due to contamination, impurities, processing on shared equipment, and the like. In an embodiment, the term "substantially free of" refers to the object including less than about 0.001, less than about 0.01, less than about 0.05, less than about 0.1, less than about 0.5, less than about 1, or less than about 2 weight %, of the chemical in the object. In particular, the term "substantially free of", in reference to an adhesive (e.g., a hot-melt glue or other adhesive used in the manufacture of stretch articles), indicates that the adhesive is intended to be excluded from, or not in contact with, an object (e.g. a stretch yarn); however, trace amounts of the adhesive might be present in/on the object due to contamination, impurities, processing on shared equipment, overspray, and the like.

As used herein, the term "substantially small amount of", in reference to a chemical, indicates that the amount of the chemical referred to is much less than the typical amount of the chemical (e.g., adhesive) used in a comparative process. In particular, the term "substantially small amount of," in reference to an adhesive indicates that the amount of adhesive referred to is much less than the typical amount of adhesive used in a comparative process. In an embodiment, the term "substantially small amount of" refers to the object including less than about 0.001, less than about 0.01, less than about 0.05, less than about 0.1, less than about 0.5, less than about 1, or less than about 2 weight %, of the chemical (e.g., adhesive) used in a comparative process.

As used herein, "elastomeric propylene based polymer" refers to an elastomeric polymer composition including a propylene backbone, as described in greater detail below. Exemplary propylene based polymers are described in US 2004/0236042 and WO05/049672 and in U.S. Pat. No. 6,881,800. Exemplary commercially available elastomeric propylene based polymers useful in making the elastomeric fibers and articles of the present disclosure include Vistamaxx™ 1100 and Vistamaxx™ 2100 by ExxonMobil Corporation. In embodiments, the elastomeric propylene based polymer may also include additional components, such as, but not limited to, an optional diene and cross-linking agent (also referred to as a coagent), to form a cross-linkable elastomeric propylene based polymer, such as described in US. Patent Application Publication No.2009/0298964 and as described in greater detail below. As used herein, the term "elastomeric propylene based polymer" refers to both types of formulations. Synthetic fibers made from the elastomeric propylene based polymers can be wound on a cylindrical core to form a supply package. Such fibers may be prepared by a melt-spinning or a dry-spinning process and can have any of a variety of cross-sections such as a round cross-section or a flat "tape-like" cross section.

Discussion

Embodiments of the present disclosure provide for stretch articles including a substrate and at least one stretch yarn bonded to the substrate material, where the stretch yarn includes an elastomeric propylene-based polymer and wherein the stretch yarn is capable of being mechanically bonded directly to the substrate surface. In embodiments, the substrate comprises at least two layers of substrate material, and the stretch yarn is positioned between two layers of substrate material and is bonded to each of the two layers. The stretch article of the present disclosure is useful for forming elastomeric portions of articles of manufacture such as laminates, fabrics, garments, and textiles including the fiber. In particular embodiments, the stretch article of the present disclosure is useful for forming elastomeric portions of wearable disposable articles such as, but not limited to, diapers, incontinence articles, and personal hygiene articles.

In embodiments, the substrate includes one or more layers of substrate material and forms a laminate article, with the stretch yarn in between layers of the laminate article. The substrate material may be made of a variety of materials, particularly nonwoven or extruded sheets including various polymeric materials. In particular embodiments the substrate material is a propylene-based material, in particular a nonwoven polypropylene-based material, which is highly compatible with the propylene-based polymer of the stretch yarn.

The stretch yarn for bonding to the stretch articles of the present disclosure may have a variety of cross sectional shapes. In an embodiment, the stretch yarn may have a traditional rounded/spherical cross section or variations of such a cross-section. In other embodiments, the stretch yarn has a flat or ribbon-like shape, such that the cross-sectional shape is elongated and/or oblong, with a rectangle or oval cross-section or variations thereof. In embodiments, the cross-sectional shape of the stretch yarn is elongated, and has a width dimension that is greater than a height dimension.

The stretch yarn used in the stretch articles of the present disclosure includes elastomeric propylene-based polymer fibers that may optionally be crosslinkable.

**Elastomeric Propylene-Based Polymer**

The elastomeric fiber of the present disclosure is made from an elastomeric propylene based polymer, as defined above. The elastomeric propylene based polymer can be a metallocene catalyzed propylene based elastomeric polymer. Exemplary commercially available elastomeric propylene based polymers suitable for use in preparing the fibers of the present disclosure include Vistamaxx™ 1100 and Vistamaxx™ 2100 by Exxon Mobil Corporation. Additional elastomeric propylene-based polymers and compositions including these polypropylene-based fibers are described below.

The elastomeric yarns, filaments and fibers in some aspects can be made from a composition including a blend of one or more elastomeric propylene-based polymers, one or more antioxidants, and one or more cross-linking agents (also referred to as coagents).

The terms "elastomeric propylene-based polymer," "propylene-based polymer," and "propylene polymer" are used interchangeably and include one or more elastomeric propylene-based polymers, one or more propylene-$\alpha$-olefin copolymers, one or more propylene-$\alpha$-olefin-diene terpolymers, and one or more propylene-diene copolymers. Blends of two or more of these polymers, copolymers and/or terpolymers are also included.

The term "elastomeric propylene-based polymer composition" refers to a composition including at least one elastomeric propylene-based polymer along with any additives which can be used to provide a melt spun filament or yarn.

The propylene-based polymer can be prepared by polymerizing propylene with one or more dienes. In at least one other specific embodiment, the propylene-based polymer can be prepared by polymerizing propylene with ethylene and/or at least one $C_4$-$C_{20}$ $\alpha$-olefin, or a combination of ethylene and at least one $C_4$-$C_{20}$ $\alpha$-olefin and one or more dienes. The one or more dienes can be conjugated or non-conjugated. Preferably, the one or more dienes are non-conjugated.

The comonomers can be linear or branched. Linear comonomers include ethylene or $C_4$-$C_8$ $\alpha$-olefin, such as ethylene, 1-butene, 1-hexene, and 1-octene. Branched comonomers include 4-methyl-1-pentene, 3-methyl-1-pentene, and 3,5,5-trimethyl-1-hexene. In one or more embodiments, the comonomer can include styrene.

Illustrative dienes can include but are not limited to 5-ethylidene-2-norbornene(ENB); 1,4-hexadiene; 5-methylene-2-norbornene (MNB); 1,6-octadiene; 5-methyl-1,4- hexadiene; 3,7-dimethyl-1,6-octadiene; 1,3-cyclopentadiene; 1,4-cyclohexadiene; vinyl norbornene (VNB); dicyclopendadiene (DCPD), and combinations thereof.

Suitable methods and catalysts for producing the propylene-based polymers are found in publications US 2004/0236042 and WO05/049672 and in U.S. Pat. No. 6,881,800. Pyridine amine complexes, such as those described in WO03/040201 are also useful to produce the propylene-based polymers useful herein. The catalyst can involve a fluxional complex, which undergoes periodic intra-molecular re-arrangement so as to provide the desired interruption of stereo regularity as in U.S. Pat. No. 6,559,262. The catalyst can be a stereorigid complex with mixed influence on propylene insertion, see Rieger EP1070087. The catalyst described in EP1614699 could also be used for the production of backbones suitable for the embodiments of the present disclosure.

Polymerization methods for preparing the elastomeric propylene-based polymers include high pressure, slurry, gas, bulk, solution phase, and combinations thereof. Catalyst systems that can be used include traditional Ziegler-Natta catalysts and single-site metallocene catalyst systems. The catalyst used may have a high isospecificity. Polymerization may be carried out by a continuous or batch process and may include the use of chain transfer agents, scavengers, or other such additives well known to those skilled in the art. The polymers may also contain additives such as flow improvers, nucleators, and antioxidants which are normally added to improve or retain resin and/or yarn properties.

One suitable catalyst is a bulky ligand transition metal catalyst. The bulky ligand contains a multiplicity of bonded atoms, for example, carbon atoms, forming a group, which may be cyclic with one or more optional hetero-atoms. The bulky ligand may be metallocene-type cyclopentadienyl derivative, which can be mono- or poly-nuclear. One or more bulky ligands may be bonded to the transition metal atom. The bulky ligand is assumed, according to prevailing scientific theory, to remain in position in the course of polymerization to provide a homogenous polymerization effect. Other ligands may be bonded or coordinated to the transition metal, optionally detachable by a cocatalyst or activator, such as a hydrocarbyl or halogen-leaving group. It is assumed that detachment of any such ligand leads to the creation of a coordination site at which the olefin monomer can be inserted into the polymer chain. The transition metal atom is a Group IV, V or VI transition metal of the Periodic Table of Elements. One suitable transition metal atom is a Group IVB atom.

Suitable catalysts include single sited catalysts (SSC). These generally contain a transition metal of Groups 3 to 10 of the Periodic Table; and at least one ancillary ligand that remains bonded to the transition metal during polymerization. The transition metal may be used in a cationic state and stabilized by a cocatalyst or activator. Examples include metallocenes of Group 4 of the Periodic table such as titanium, hafnium or zirconium which are used in polymerization in the $d^0$ mono-valent cationic state and have one or two ancillary ligands as described in more detail hereafter. Some features of such catalysts useful for coordinating polymerization include a ligand capable of abstraction and a ligand into which the ethylene (olefinic) group can be inserted.

The metallocene can be used with a cocatalyst, which may be alumoxane such as methylalumoxane having an average degree of oligomerization of 4 to 30 as determined by vapor pressure osmometry. Alumoxane may be modified to provide solubility in linear alkanes or be used in a slurry but is generally used from a toluene solution. Such solutions may include unreacted trialkyl aluminum and the alumoxane concentration is generally indicated as mol Al per liter, which figure includes any trialkyl aluminum which has not so reacted to form an oligomer. The alumoxane, when used as cocatalyst, is generally used in molar excess, at a mol ratio of about 50 or more, including about 100 or more, about 1000 or less, and about 500 or less, relative to the transition metal.

The SSC may be selected from among a broad range, of available SSC's, to suit the type of polymer being made and the process window associated therewith in such a way that the polymer is produced under the process conditions at an activity of at least about 40,000 gram polymer per gram SSC (such as a metallocene), such as at least about 60,000 including in excess of about 100,000 gram polymer per gram SSC. By enabling the different polymers to be produced in different operating windows with an optimized catalyst selection, the SSC and any ancillary catalyst components can be used in small quantities, with optionally also using small amounts of scavengers. A catalyst killer can be used in equally small amounts and the various cost-effective methods can then be introduced to allow the non-polar solvent to be recycled and subjected to treatment to remove polar contaminants before re-use in the polymerization reactor(s).

The metallocene may be also be used with a cocatalyst that is a non- or weakly coordinated anion (the term non-coordinating anion as used herein includes weakly coordinated anions). The coordination should be sufficiently weak in any event, as evidenced by the progress of polymerization, to permit the insertion of the unsaturated monomer component. The non-coordinating anion may be supplied and reacted with the metallocene in any of the manners described in the art.

The precursor for the non-coordinating anion may be used with a metallocene supplied in a reduced valency state. The precursor may undergo a redox reaction. The precursor may be an ion pair of which the precursor cation is neutralized and/or eliminated in some manner. The precursor cation may be an ammonium salt. The precursor cation may be a triphenylcarbonium derivative.

The non-coordinating anion can be a halogenated, tetraaryl-substituted Group 10-14 non-carbon element-based anion, especially those that are have fluorine groups substituted for hydrogen atoms on the aryl groups, or on alkyl substituents on those aryl groups.

Effective Group 10-14 element cocatalyst complexes may be derived from an ionic salt including a 4-coordinate Group 10-14 element anionic complex, where $A^-$ can be represented as

$$[(M)Q_1Q_2...Q_i]^-$$

where M is one or more Group 10-14 metalloid or metal, such as boron or aluminum, and each Q is a ligand effective for providing electronic or steric effects rendering $[(M') Q_1Q_2 ... Q_i]^-$ suitable as a non-coordinating anion as that is understood in the art, or a sufficient number of Q are such that $[(M') Q_1Q_2... Q Q_i]^-$ as a whole is an effective non-coordinating or weakly coordinating anion. Exemplary Q substituents specifically include fluorinated aryl groups, such as perfluorinated aryl groups, and include substituted Q groups having substituents additional to the fluorine substitution, such as fluorinated hydrocarbyl groups. Exemplary fluorinated aryl groups include phenyl, biphenyl, naphthyl and derivatives thereof.

The non-coordinating anion may be used in approximately equimolar amounts relative to the transition metal component, such as at least about 0.25, including about 0.5 and about 0.8 and no more than about 4, or about 2 or about 1.5.

Representative metallocene compounds can have the formula:

$$L^A L^B L^C_i MDE$$

where, $L^A$ is a substituted cyclopentadienyl or heterocyclopentadienyl ancillary ligand $\pi$-bonded to M; $L^B$ is a member of the class of ancillary ligands defined for $L^A$, or is J, a hetero-atom ancillary ligand $\sigma$-bonded to M; the $L^A$ and $L^B$ ligands may be covalently bridged together through a Group 14 element linking group; $L^C_i$ is an optional neutral, non-oxidizing ligand having a dative bond to M (i equals 0 to 3); M is a Group 4 or 5 transition metal; and, D and E are independently mono-anionic labile ligands, each having a a-bond to M, optionally bridged to each other or $L^A$ or $L^B$. The mono-anionic ligands are displaceable by a suitable activator to permit insertion of a polymerizable monomer or macro-monomer can insert for coordination polymerization on the vacant coordination site of the transition metal component.

Representative non-metallocene transition metal compounds usable as SSC's also include tetrabenzyl zirconium, tetra bis(trimethylsiylmethyl) zirconium, oxotris (trimethlsilylmethyl) vanadium, tetrabenzyl hafnium, tetrabenzyl titanium, bis(hexamethyl disilazido)dimethyl titanium, tris(trimethyl silyl methyl) niobium dichloride, and tris(trimethylsilylmethyl) tantalum dichloride.

Additional organometallic transition metal compounds suitable as olefin polymerization catalysts in accordance with the invention will be any of those Group 3-10 that can be converted by ligand abstraction into a catalytically active cation and stabilized in that active electronic state by a non-coordinating or weakly coordinating anion sufficiently labile to be displaced by an olefinically unsaturated monomer such as ethylene.

Other useful catalysts include metallocenes which are biscyclopentadienyl derivatives of a Group IV transition metal, such as zirconium or hafnium. These may be derivatives containing a fluorenyl ligand and a cyclopentadienyl ligand connected by a single carbon and silicon atom. The Cp ring may be unsubstituted and/or the bridge contains alkyl substituents, suitably alkylsilyl substituents to assist in the alkane solubility of the metallocene such as those disclosed in PCT published applications WO00/24792 and WO00/24793. Other possible metallocenes include those in PCT published application WO01/58912.

Other suitable metallocenes may be bisfluorenyl derivatives or unbridged indenyl derivatives which may be substituted at one or more positions on the fused ring with moieties which have the effect of increasing the molecular weight and so indirectly permit polymerization at higher temperatures.

The total catalyst system may additionally include one or more organometallic compounds as scavenger. Such compounds are meant to include those compounds effective for removing polar impurities from the reaction environment and for increasing catalyst activity. Impurities can be inadvertently introduced with any of the polymerization reaction components, particularly with solvent, monomer and catalyst feed, and adversely affect catalyst activity and stability. It can result in decreasing or even elimination of catalytic activity, particularly when ionizing anion pre-cursors activate the catalyst system. The impurities, or catalyst poisons include water, oxygen, polar organic compounds, metal impurities, etc. Steps can be taken to remove these poisons before introduction of such into the reaction vessel, for example, by chemical treatment or careful separation techniques after or during the synthesis or preparation of the various components, but some minor amounts of organometallic compound will still normally be used in the polymerization process itself.

Typically organometallic compounds can include the Group-13 organometallic compounds disclosed in U.S. Patent Nos. 5,153,157 and 5,241,025 and PCT publications WO91/09882, WO94/03506, WO93/14132, and WO95/07941. Suitable compounds include triethyl aluminum, triethyl borane, triisobutyl aluminum, tri-n-octyl aluminum, methylalumoxane, and isobutyl alumoxane. Alumoxane also may be used in scavenging amounts with other means of activation, e.g., methylalumoxane and tri-isobutylaluminoxane with boron-based activators. The amount of such compounds to be used with catalyst compounds is minimized during polymerization reactions to that amount effective to enhance activity (and

with that amount necessary for activation of the catalyst compounds if used in a dual role) since excess amounts may act as catalyst poisons.

The propylene-based polymer can have an average propylene content on a weight percent basis of about 60 wt % to about 99.7 wt %, including about 60 wt % to about 99.5 wt %, about 60 wt % to about 97 wt % and about 60 wt % to about 95 wt % based on the weight of the polymer. In one aspect, the balance may include one or more other $\alpha$-olefins or one or more dienes. In other embodiments, the content can be about 80 wt % to about 95 wt % propylene, about 83 wt % to about 95 wt % propylene, about 84 wt % to about 95 wt % propylene, and about 84 wt % to about 94 wt % propylene based on the weight of the polymer. The balance of the propylene-based polymer optionally comprises a diene and/or one or more $\alpha$-olefins. The $\alpha$-olefin may include ethylene, butene, hexene or octene. When two $\alpha$-olefins are present, they may include any combination such as ethylene and one of butene, hexene or octene. The propylene-based polymer comprises about 0.2 wt % to about 24 wt %, of a non-conjugated diene based on the weight of the polymer, including about 0.5 wt % to about 12 wt %, about 0.6 wt % to about 8 wt %, and about 0.7 wt % to about 5 wt %. In other embodiments, the diene content can be about 0.2 wt % to about 10 wt %, including about 0.2 to about 5 wt %, about 0.2 wt % to about 4 wt %, about 0.2 wt % to about 3.5 wt %, about 0.2 wt % to about 3.0 wt %, and about 0.2 wt % to about 2.5 wt % based on the weight of the polymer. In one or more embodiments above or elsewhere herein, the propylene-based polymer comprises ENB in an amount of about 0.5 to about 4 wt %, including about 0.5 to about 2.5 wt %, and about 0.5 to about 2.0 wt %.

In other embodiments, the propylene-based polymer includes propylene and diene in one or more of the ranges described above with the balance comprising one or more $C_2$ and/or $C_4$-$C_{20}$ α-olefins. In general, this will amount to the propylene-based polymer including about 5 to about 40 wt % of one or more $C_2$ and/or $C_4$-$C_{20}$ α-olefins based the weight of the polymer. When $C_2$ and/or a $C_4$-$C_{20}$ α-olefins are present the combined amounts of these olefins in the polymer may be about 5 wt % or greater and falling within the ranges described herein. Other suitable ranges for the one or more α-olefins include about 5 wt % to about 35 wt %, including about 5 wt % to about 30 wt %, about 5 wt % to about 25 wt %, about 5 wt% to about 20 wt %, about 5 to about 17 wt % and about 5 wt % to about 16 wt %.

The propylene-based polymer can have a weight average molecular weight (Mw) of about 5,000,000 or less, a number average molecular weight (Mn) of about 3,000,000 or less, a z-average molecular weight (Mz) of about 10,000,000 or less, and a g' index of about 0.95 or greater measured at the weight average molecular weight (Mw) of the polymer using isotactic polypropylene as the baseline, all of which can be determined by size exclusion chromatography, e.g., 3D SEC, also referred to as GPC-3D as described herein.

In one or more embodiments above or elsewhere herein, the propylene-based polymer can have a Mw of about 5,000 to about 5,000,000 g/mole, including a Mw of about 10,000 to about 1,000,000, a Mw of about 20,000 to about 500,000 and a Mw of about 50,000 to about 400,000, wherein Mw is determined as described herein.

In one or more embodiments above or elsewhere herein, the propylene-based polymer can have a Mn of about 2,500 to about 2,500,000 g/mole, including a Mn of about 5,000 to about 500,000, a Mn of about 10,000 to about 250,000, and a Mn of about 25,000 to about 200,000, wherein Mn is determined as described herein.

In one or more embodiments above or elsewhere herein, the propylene-based polymer can have a Mz of about 10,000 to about 7,000,000 g/mole, including a Mz of about 50,000 to about 1,000,000, a Mz of about 80,000 to about 700,000, and a Mz of about 100,000 to about 500,000, wherein Mz is determined as described herein.

The molecular weight distribution index (MWD= (Mw/Mn)), sometimes referred to as a "polydispersity index" (PDI), of the propylene-based polymer can be about 1.5 to 40. The MWD can have an upper limit of about 40, or about 20, or about 10, or about 5, or about 4.5, and a lower limit of about 1.5, or about 1.8, or about 2.0. The MWD of the propylene-based polymer may be about 1.8 to 5 and including about 1.8 to 3. Techniques for determining the molecular weight (Mn and Mw) and molecular weight distribution (MWD) are well known in the art and can be found in U.S. Patent No. 4,540,753 and references cited therein, in Macromolecules, 1988, volume 21, p 3360 (Verstrate et al), and in accordance with the procedures disclosed in U.S. Patent No. 6,525,157, column 5, lines 1-44.

The propylene-based polymer can have a g' index value of about 0.95 or greater, including about 0.98 or greater and about 0.99 or greater wherein g' is measured at the Mw of the polymer using the intrinsic viscosity of isotactic polypropylene as the baseline. For use herein, the g' index is defined as:

$$g' = \eta_b / \eta_l$$

where $\eta_b$ is the intrinsic viscosity of the propylene-based polymer and $\eta_l$ is the intrinsic viscosity of a linear polymer of the same viscosity-averaged molecular weight ($M_v$) as the propylene-based polymer. $\eta_l = KM_v^\alpha$, K and α were measured values for linear polymers and should be obtained on the same instrument as the one used for the g' index measurement.

The propylene-based polymer can have a density of about 0.85 g/cm$^3$ to about 0.92 g/cm$^3$, including about 0.87 g/cm$^3$ to 0.90 g/cm$^3$ and about 0.88 g/cm$^3$ to about 0.89 g/cm$^3$ at about room temperature as measured per the ASTM D-1505 test method.

The propylene-based polymer can have a melt flow rate MFR, about 2.16 kg weight (230° C), equal to or greater than about 0.2 g/10 min as measured according to the ASTM D-I 238(A) test method as modified (described below). The MFR (2.16 kg (230° C) may be about 0.5 g/10 min to about 200 g/10 min including about 1 g/10 min to about 100 g/10 min. The propylene-based polymer may have an MFR of about 0.5 g/10 min to about 200 g/10 min, including about 2 g/10 min to about 30 g/10 min, about 5 g/10 min to about 30 g/10 min, about 10 g/10 min to about 30 g/10 min, about 10 g/10 min to about 25 g/10 min, and about 2 g/10 min to about 10 g/10 min.

The propylene-based polymer can have a Mooney viscosity ML (1+4) 125° C, as determined according to ASTM D1646, of less than about 100, such as less than about 75, including less than about 60 and less than about 30.

The propylene-based polymer can have a heat of fusion (Hf) determined according to the DSC procedure described later, which is greater than or equal to about 0.5 Joules per gram (J/g), and can be about 80 J/g, including about 75 J/g, about 70 J/g, about 60 J/g, about 50 J/g, and about 35J/g. The propylene-based polymer may have a heat of fusion that is greater than or equal to about 1 J/g, including greater than or equal to about 5 J/g. In another embodiment, the propylene-based polymer can have a heat of fusion (Hf), which can be about 0.5 J/g to about 75 J/g, including about 1 J/g to about 75 J/g and about 0.5 J/g to about 35 J/g.

Suitable propylene-based polymers and compositions can be characterized in terms of both their melting points (Tm) and heats of fusion, which properties can be influenced by the presence of comonomers or steric irregularities that hinder the formation of crystallites by the polymer chains. In one or more embodiments, the heat of fusion can have a lower limit of about 1.0 J/g, or 1.5 J/g, or about 3.0 J/g, or about 4.0 J/g, or about 6.0 J/g, or about 7.0 J/g, to an upper limit of about 30 J/g, or about 35 J/g, or about 40 J/g, or about 50 J/g, or about 60 J/g or about 70 J/g, or about 75 J/g, or about 80 J/g.

The crystallinity of the propylene-based polymer can also be expressed in terms of percentage of crystallinity (i.e., % crystallinity). In one or more embodiments above or elsewhere herein, the propylene-based polymer has a % crystallinity of about 0.5% to 40%, including about 1 % to 30% and about 5% to 25% wherein % crystallinity is determined according to the DSC procedure described below. In another embodiment, the propylene-based polymer may have a crystallinity of less than about 40%, including about 0.25% to about 25%, from about 0.5% to about 22%, and from about 0.5% to about 20%. As disclosed above, the thermal energy for the highest order of polypropylene is estimated at about 189 J/g (i.e., 100% crystallinity is equal to 209 J/g.).

In addition to this level of crystallinity, the propylene-based polymer may have a single broad melting transition. Also, the propylene-based polymer can show secondary melting peaks adjacent to the principal peak, but for purposes herein, such secondary melting peaks are considered together as a single melting point, with the highest of these peaks (relative to baseline as described herein) being considered the melting point of the propylene-based polymer.

The propylene-based polymer may have a melting point (measured by DSC) of equal to or less than about 100° C, including less than about 90° C, less than about 80° C, and less than or equal to about 75° C, including the range from about 25° C to about 80° C, about 25° C to about 75° C, and about 30° C to about 65° C.

The Differential Scanning Calorimetry (DSC) procedure can be used to determine heat of fusion and melting temperature of the propylene-based polymer. The method is as follows: about 0.5 grams of polymer is weighed out and pressed to a thickness of about 15-20 mils (about 381-508 microns) at about 140° C -150° C, using a "DSC mold" and Mylar as a backing sheet. The pressed pad is allowed to cool to ambient temperature by hanging in air (the Mylar is not removed). The pressed pad is annealed at room temperature (23-25° C) for about 8 days. At the end of this period, an about 15-20 mg disc is removed from the pressed pad using a punch die and is placed in a 10 microliter aluminum sample pan. The sample is placed in a Differential Scanning Calorimeter (Perkin Elmer Pyris 1 Thermal Analysis System) and is cooled to about -100° C. The sample is heated at 10° C/min to attain a final temperature of about 165° C. The thermal output, recorded as the area under the melting peak of the sample, is a measure of the heat of fusion and can be expressed in Joules per gram of polymer and is automatically calculated by the Perkin Elmer System. The melting point is recorded as the temperature of the greatest heat absorption within the range of melting of the sample relative to a baseline measurement for the increasing heat capacity of the polymer as a function of temperature.

The propylene-based polymer can have a triad tacticity of three propylene units, as measured by 13C NMR of about 75% or greater, about 80% or greater, about 82% or greater, about 85% or greater, or about 90% or greater. In an embodiment, the triad tacticity can be about 50 to about 99%, about 60 to about 99%, about 75 to about 99%, about 80 to about 99%; and in other embodiments about 60 to about 97%. Triad tacticity is well-known in the art and may be determined by the methods described in U.S. Patent Application Publication No. 2004/0236042.

The elastomeric propylene-based polymer can include a blend of two propylene-based polymers differing in the olefin content, the diene content, or both.

In one or more embodiments above or elsewhere herein, the propylene-based polymer can include a propylene based elastomeric polymer produced by random polymerization processes leading to polymers having randomly distributed irregularities in stereoregular propylene propagation. This is in contrast to block copolymers in which constituent parts of the same polymer chains are separately and sequentially polymerized.

The propylene-based polymers can also include copolymers prepared according the procedures in WO 02/36651. Likewise, the propylene-based polymer can include polymers consistent with those described in WO 03/040201, WO 03/040202, WO 03/040095, WO 03/040201, WO 03/040233, and/or WO 03/040442. Additionally, the propylene-based polymer can include polymers consistent with those described in EP 1 233 191, and U.S. Pat. No. 6,525,157, along with suitable propylene homo- and copolymers described in U.S. Pat. No. 6,770,713 and U.S. Patent Application Publication 2005/215964. The propylene-based polymer can also include one or more polymers consistent with those described in EP 1 614 699 or EP 1 017 729.

**Grafted (Functionalized) Backbone**

In one or more embodiments, the propylene-based polymer can be grafted (i.e. "functionalized") using one or more grafting monomers. As used herein, the term "grafting" denotes covalent bonding of the grafting monomer to a polymer chain of the propylene-based polymer.

The grafting monomer can be or include at least one ethylenically unsaturated carboxylic acid or acid derivative, such as an acid anhydride, ester, salt, amide, imide, and acrylates, among others. Illustrative monomers include, but are not limited to, acrylic acid, methacrylic acid, maleic acid, fumaric acid, itaconic acid, citraconic acid, mesaconic acid, maleic anhydride, 4-methyl cyclohexene-1,2-dicarboxylic acid anhydride, bicyclo(2,2,2)octene-2,3-dicarboxylic acid anhydride, 1,2,3,4,5,8,9,10-octahydronaphthalene-2,3-dicarboxylic acid anhydride, 2-oxa-1,3-diketospiro(4,4)nonene, bicycle (2,2,1)heptene-2,3-dicarboxylic acid anhydride, maleopimaric acid, tetrahydrophthalic anhydride, norbornene-2,3-dicarboxylic acid anhydride, nadic anhydride, methyl nadic anhydride, himic anhydride, methyl himic anhydride, and 5-methylbicyclo(2,2,1)heptene-2,3-dicarboxylic acid anhydride. Other suitable grafting monomers include methyl acrylate and higher alkyl acrylates, methyl methacrylate and higher alkyl methacrylates, acrylic acid, methacrylic acid, hydroxy-methyl methacrylate, hydroxyl-ethyl methacrylate and higher hydroxyalkyl methacrylates and glycidyl methacrylate. Maleic anhydride is a preferred grafting monomer.

In one or more embodiments, the grafted propylene based polymer comprises about 0.5 to about 10 wt % ethylenically unsaturated carboxylic acid or acid derivative, including about 0.5 to about 6 wt %, about 0.5 to about 3 wt %; in other embodiments about 1 to about 6 wt %, and about 1 to about 3 wt %. Where the graft monomer is maleic anhydride, the maleic anhydride concentration in the grafted polymer may be about 1 to about 6 wt. %, including about 0.5 wt. % or about 1.5 wt. % as a minimum.

Styrene and derivatives thereof such as paramethyl styrene, or other higher alkyl substituted styrenes such as t-butyl styrene can be used as a charge transfer agent in presence of the grafting monomer to inhibit chain scissioning. This allows further minimization of the beta scission reaction and the production of a higher molecular weight grafted polymer (MFR=1.5).

## Preparing Grafted Propylene-Based Polymers

A grafted propylene-based polymer can be prepared using conventional techniques. For example, the graft polymer can be prepared in solution, in a fluidized bed reactor, or by melt grafting. A grafted polymer can be prepared by melt blending in a shear-imparting reactor, such as an extruder reactor. Single screw or twin screw extruder reactors such as co-rotating intermeshing extruder or counter-rotating non-intermeshing extruders but also co-kneaders such as those sold by Buss are useful for this purpose.

The grafted polymer can be prepared by melt blending an ungrafted propylene-based polymer with a free radical generating catalyst, such as a peroxide initiator, in the presence of a grafting monomer. One suitable sequence for the grafting reaction includes melting the propylene-based polymer, adding and dispersing the grafting monomer, introducing peroxide and venting the unreacted monomer and by-products resulting from the peroxide decomposition. Other sequences can include feeding the monomers and the peroxide predissolved in a solvent.

Illustrative peroxide initiators include but are not limited to: diacyl peroxides such as benzoyl peroxide; peroxyesters such as tert-butylperoxy benzoate, tert-butylperoxy acetate, O,O-tert-butyl-O-(2-ethylhexyl)monoperoxy carbonate; peroxyketals such as n-butyl-4,4-di-(tert-butyl peroxy) valerate; and dialkyl peroxides such as 1,1-bis(tertbutylperoxy) cyclohexane, 1,1-bis(tert-butylperoxy)-3,3,5-trimethylcyclohexane, 2,2-bis(tert-butylperoxy)butane, dicumylperoxide, tert-butylcumylperoxide, Di-(2-tert-butylperoxyisopropyl-(2))benzene, di-tert-butylperoxide (DTBP), 2,5- dimethyl-2,5-di(tert-butylperoxy)hexane, 2,5-dimethyl-2,5- di(tert-butylperoxy)hexyne, 3,3,5,7,7-pentamethyl 1,2,4-trioxepane; among others and combinations thereof.

## Polyolefinic Thermoplastic Resin

The term "polyolefinic thermoplastic resin" as used herein refers to any material that is not a "rubber" and that is a polymer or polymer blend having a melting point of 70°C or more and considered by persons skilled in the art as being thermoplastic in nature, e.g., a polymer that softens when exposed to heat and returns to its original condition when cooled to room temperature. The polyolefinic thermoplastic resin can contain one or more polyolefins, including polyolefin homopolymers and polyolefin copolymers. Except as stated otherwise, the term "copolymer" means a polymer derived from two or more monomers (including terpolymers, tetrapolymers, etc.), and the term "polymer" refers to any carbon-containing compound having repeat units from one or more different monomers.

Illustrative polyolefins can be prepared from monoolefin monomers including, but are not limited to, monomers having 2 to 7 carbon atoms, such as ethylene, propylene, 1-butene, isobutylene, 1-pentene, 1-hexene, 1-octene, 3-methyl-1-pentene, 4-methyl-1-pentene, 5-methyl-1-hexene, mixtures thereof and copolymers thereof with (meth)acrylates and/or vinyl acetates. The polyolefinic thermoplastic resin component is unvulcanized or non crosslinked.

The polyolefinic thermoplastic resin may contain polypropylene. The term "polypropylene" as used herein broadly means any polymer that is considered a "polypropylene" by persons skilled in the art and includes homo, impact, and random polymers of propylene. The polypropylene used in the compositions described herein has a melting point above about 110°C, includes at least about 90 wt % propylene units, and contains isotactic sequences of those units. The polypropylene can also include atactic sequences or s syndiotactic sequences, or both. The polypropylene can also include essentially syndiotactic sequences such that the melting point of the polypropylene is above about 110°C. The polypropylene can either derive exclusively from propylene monomers (i.e., having only propylene units) or derive from mainly propylene (more than about 80% propylene) with the remainder derived from olefins, such as ethylene, and/or $C_4$-$C_{10}$ $\alpha$-olefins. Certain polypropylenes have a high MFR (e.g., from a low of about 10, or about 15, or about 20g/10min to a high of about 25 or about 30g/10min.) Others have a lower MFR, e.g., "fractional" polypropylenes which have an MFR less than about 1.0. Those with high MFR can be useful for ease of processing or compounding.

A polyolefinic thermoplastic resin may be or include isotactic polypropylene. The polyolefinic thermoplastic resin may contain one or more crystalline propylene homopolymers or copolymers of propylene having a melting temperature greater than about 105°C as measured by DSC. Exemplary copolymers of propylene include, but are not limited to, terpolymers of propylene, impact copolymers of propylene, random polypropylene and mixtures thereof. The comonomers may have 2 carbon atoms, or 4 to 12 carbon atoms, such as ethylene. Such polyolefinic thermoplastic resin and methods for making the same are described in U.S. Pat. No. 6,342,565, which is incorporated herein by reference. The term "random polypropylene" as used herein broadly means a copolymer of propylene having up to about 9 wt %, such as about 2 wt % to 8 wt % of an alpha olefin comonomer. An $\alpha$-olefin comonomer may have 2 carbon atoms, or 4 to 12 carbon atoms.

A random polypropylene may have a 1% secant modulus of about 100 kPsi to about 1380 MPA (200 kPsi), as measured according to ASTM D790A. The 1% secant modulus can be about 965 MPa (140 kPsi) to 1170 MPa (170 kPsi), as measured according to ASTM D790A, including from about 965 MPa (140 kPsi) to 1100 MPa (160 kPsi) or having a low of about 689 (100), about 758 (110), or about 862 MPa (125 kPsi) to a high of about 1000 (145), about 1100 (160), or about 1210 MPa (175 kPsi), as measured according to ASTM D790A.

Random polypropylene can have a density of about 0.85 to about 0.95 g/cm$^3$, as measured by ASTM D79, including a density of about 0.89 g/cm$^3$ to 0.92 g/cm$^3$, or having a low of about 0.85, 0.87, or 0.89 g/cm$^3$ to a high of about 0.90, 0.91, 0.92 g/cm$^3$, as measured by ASTM D792.

**Additional Elastomeric Component**

The elastomeric polypropylene-based polymer composition can optionally include one or more additional elastomeric components. The additional elastomeric component can be or include one or more ethylene-propylene copolymers (EP). The ethylene-propylene polymer (EP) is non-crystalline, e.g., atactic or amorphous, but the EP may be crystalline (including "semi-crystalline"). The crystallinity of the EP may be derived from the ethylene, which can be determined by a number of published methods, procedures and techniques. The crystallinity of the EP can be distinguished from the crystallinity of the propylene-based polymer by removing the EP from the composition and then measuring the crystallinity of the residual propylene-based polymer. Such crystallinity measured is usually calibrated using the crystallinity of polyethylene and related to the comonomer content. The percent crystallinity in such cases is measured as a percentage of polyethylene crystallinity and thus the origin of the crystallinity from ethylene is established.

In one or more embodiments, the EP can include one or more optional polyenes, including particularly a diene; thus, the EP can be an ethylene-propylene-diene (commonly called "EPDM"). The optional polyene is considered to be any hydrocarbon structure having at least two unsaturated bonds wherein at least one of the unsaturated bonds is readily incorporated into a polymer. The second bond may partially take part in polymerization to form long chain branches but preferably provides at least some unsaturated bonds suitable for subsequent curing or vulcanization inpost polymerization processes. Examples of EP or EPDM copolymers include V722, V3708P, MDV 91-9, V878 that are commercially available under the trade name VISTALON from ExxonMobil Chemicals. Several commercial EPDM are available from DOW under the trade names Nordel IP and MG grades.). Certain rubber components (e.g., EPDMs, such as VISTALON 3666) include additive oil that is preblended before the rubber component is combined with the thermoplastic. The type of additive oil utilized will be that customarily used in conjunction with a particular rubber component.

Examples of the optional polyenes include, but are not limited to, butadiene, pentadiene, hexadiene (e.g., 1,4-hexadiene), heptadiene (e.g., 1,6-heptadiene), octadiene (e.g., 1,7-octadiene), nonadiene (e.g., 1,8-nonadiene), decadiene (e.g., 1,9-decadiene), undecadiene (e.g., 1,10-undecadiene), dodecadiene (e.g., 1,11-dodecadiene), tridecadiene (e.g., 1,12-tridecadiene),tetradecadiene (e.g., 1,13-tetradecadiene), pentadecadiene, hexadecadiene, heptadecadiene, octadecadiene, nonadecadiene, icosadiene, heneicosadiene, docosadiene, tricosadiene, tetracosadiene, pentacosadiene, hexacosadiene, heptacosadiene, octacosadiene, nonacosadiene, triacontadiene, and polybutadienes having a molecular weight (Mw) of less than 1000 g/mol. Examples of straight chain acyclic dienes include, but are not limited to, 1,4-hexadiene and 1,6-octadiene. Examples of branched chain acyclic dienes include, but are not limited to 5-methyl-1,4-hexadiene, 3,7-dimethyl-1,6-octadiene, and 3,7-dimethyl-1,7-octadiene. Examples of single ring alicyclic dienes include, but are not limited to 1,4-cyclohexadiene, 1,5-cyclooctadiene, and 1,7-cyclododecadiene. Examples of multiring alicyclic fused and bridged ring dienes include, but are not limited to, tetrahydroindene; norbornadiene; methyltetrahydroindene; dicyclopentadiene; bicyclo(2,2,1)hepta-2,5-diene; and alkenyl-, alkylidene-, cycloalkenyl-, and cylcoalkyliene norbornenes [including, e.g., 5-methylene-2-norbornene, 5-ethylidene- 2-norbornene, 5-propenyl-2-norbornene, 5-isopropylidene-2-norbornene, 5-(4-cyclopentenyl)-2-norbornene, 5-cyclohexylidene-2-norbornene, and 5-vinyl-2-norbornene]. Examples of cycloalkenyl-substituted alkenes include, but are not limited to, vinyl cyclohexene, allyl cyclohexene, vinylcyclooctene, 4-inylcyclohexene, allyl cyclodecene, vinylcyclododecene, and tetracyclododecadiene.

In another embodiment, the additional elastomeric component can include, but is not limited to, styrene/butadiene rubber (SBR), styrene/isoprene rubber (SIR), styrene/ isoprene/butadiene rubber (SIBR), styrene-butadienestyrene block copolymer (SBS), hydrogenated styrenebutadiene-styrene block copolymer (SEBS), hydrogenated styrenebutadiene block copolymer (SEB), styrene-isoprenestyrene block copolymer (SIS), styrene-isoprene block copolymer (SI), hydrogenated styrene-isoprene block copolymer (SEP), hydrogenated styrene-isoprene-styrene block copolymer (SEPS), styrene-ethylene/butylene-ethylene block copolymer (SEBE), styrene-ethylene-styrene block copolymer (SES), ethylene-ethylene/butylene block copolymer (EEB), ethylene-ethylene/butylene/styrene block copolymer (hydrogenated BR-SBR block copolymer), styrene- ethylene/butylene-ethylene block copolymer (SEBE), ethylene-ethylene/butylene-ethylene block copolymer (EEBE), polyisoprene rubber, polybutadiene rubber, isoprene butadiene rubber (IBR), polysulfide, nitrile rubber, propylene oxide polymers, star-branched butyl rubber and halogenated star-branched butyl rubber, brominated butyl rubber, chlorinated butyl rubber, star-branched polyisobutylene rubber, star-branched brominated butyl (polyisobutylene/isoprene copolymer) rubber; poly(isobutylene-co-alkylstyrene), suitable isobutylene/methylstyrene copolymers such as isobutylene/ meta-bromomethylstyrene, isobutylene/bromomethylstyrene, isobutylene/chloromethylstyrene, halogenated isobutylene cyclopentadiene, and isobutylene/chloromethylstyrene and mixtures thereof. The additional elastomeric components include hydrogenated styrene-butadienestyrene block copolymer (SEBS), and hydrogenated styreneisoprene- styrene block copolymer (SEPS).

The additional elastomeric component can also be or include natural rubber. Natural rubbers are described in detail by Subramaniam in RUBBER TECHNOLOGY 179- 208 (1995). Suitable natural rubbers can be selected from the group consisting of Malaysian rubber such as SMR CV, SMR 5, SMR 10, SMR 20, and SMR 50 and mixtures thereof, wherein the natural rubbers have a Mooney viscosity at about 100° C. (ML 1 +4) of about 30 to 120, including about 40 to 65. The Mooney viscosity test referred to herein is in accordance with ASTM D-1646.

The additional elastomeric component can also be or include one or more synthetic rubbers. Suitable commercially available synthetic rubbers include NATSYN™ (Goodyear Chemical Company), and BUDENE™ 1207 or BR 1207 (Goodyear Chemical Company). A suitable rubber is high cis-polybutadiene (cis-BR). By "cis-polybutadiene" or "high cis-polybutadiene", it is meant that 1,4-cis polybutadiene is used, wherein the amount of cis component is at least about 95%. An example of high cis-polybutadiene commercial products used in the composition BUDENE™ 1207.

The additional elastomeric component can be present up to about 50 % by weight (50 phr), up to about 40% by weight (40 phr) or up to about 30% by weight (30 phr). In one or more embodiments, the amount of the additional rubber component can have a low of about 1 % by weight (1), or about 7% by weight (7), or about 17% by weight (17), or about 20 % by weight (20 phr) to a high of about 25% by weight (25), about 35% by weight (35), or about 50% by weight (50) phr. **Additive Oil**

The elastomeric propylene-based polymer composition can optionally include one or more additive oils. Such "additive oils" should not be confused with "finishing oils" as used in the present disclosure. The "additive oils," as used herein, refer to such oils added during preparation of the elastomeric propylene based polymer, as opposed to a finishing oil, which, as defined above, is applied to the surface of a formed fiber. The term "additive oil" includes both "process oils" and "extender oils." For example, "additive oil" may include hydrocarbon oils and plasticizers, such as organic esters and synthetic plasticizers. Many additive oils are derived from petroleum fractions, and have particular ASTM designations depending on whether they fall into the class of paraffinic, naphthenic, or aromatic oils. Other types of additive oils include mineral oil, alpha olefinic synthetic oils, such as liquid polybutylene, e.g., products sold under the trademark Parapol®. Additive oils other than petroleum based oils can also be used, such as oils derived from coal tar and pine tar, as well as synthetic oils, e.g., polyolefin materials (e.g., SpectaSyn™ and Elevast™, both supplied by ExxonMobil Chemical Company.

It is well-known in the art which type of oil should be used with a particular rubber, as well as suitable amounts (quantity) of oil. The additive oil can be present in amounts of about 5 to about 300 parts by weight per 100 parts by weight of the blend of the rubber and thermoplastic components. The amount of additive oil may also be expressed as about 30 to 250 parts or about 70 to 200 parts by weight per 100 parts by weight of the rubber component. Alternatively, the quantity of additive oil can be based on the total rubber content, and defined as the ratio, by weight, of additive oil to total rubber and that amount may in certain cases be the combined amount of process oil and extender oil. The ratio may be, for example, about 0 to about 4.0/1. Other ranges, having any of the following lower and upper limits, may also be utilized: a lower limit of about 0.1/1, or 0.6/1, or about 0.8/1, or about 1.0/1, or about 1.2/1, or about 1.5/1, or about 1.8/1, or about 2.0/1, or about 2.5/1; and an upper limit (which may be combined with any of the foregoing lower limits) of about 4.0/1, or about 3.8/1, or about 3.5/1, or about 3.2/1, or about 3.0/1, or about 2.8/1. Larger amounts of additive oil can be used, although the deficit is often reduced physical strength of the composition, or oil weeping, or both.

Polybutene oils are suitable. Exemplary polybutene oils have an Mn of less than about 15,000, and include homopolymer or copolymer of olefin derived units having 3 to 8 carbon atoms and more preferably 4 to 6 carbon atoms. The polybutene may be a homopolymer or copolymer of a $C_4$ raffinate. Exemplary low molecular weight polymers termed "polybutene" polymers is described in, for example, SYNTHETIC LUBRICANTS AND HIGH-PERFORMANCE FUNCTIONAL FLUIDS 357-392 (Leslie R. Rudnick & Ronald L. Shubkin, ed., Marcel Dekker 1999) (hereinafter "polybutene processing oil" or "polybutene").

The polybutene processing oil can be a copolymer having at least isobutylene derived units, and optionally 1-butene derived units, and/or 2-butene derived units. The polybutene can be a homopolymer if isobutylene, or a copolymer of isobutylene and 1-butene or 2-butene, or a terpolymer of isobutylene and 1-butene and 2-butene, wherein the isobutylene derived units are about 40 to 100 wt % of the copolymer, the 1-butene derived units are about 0 to 40 wt % of the copolymer, and the 2-butene derived units are about 0 to 40 wt% of the copolymer. The polybutene can be a copolymer or terpolymer wherein the isobutylene derived units are about 40 to 99 wt % of the copolymer, the 1-butene derived units are about 2 to 40 wt % of the copolymer, and the 2-butene derived units are about 0 to 30 wt % of the copolymer. The polybutene may also be a terpolymer of the three units, wherein the isobutylene derived units are about 40 to 96 wt % of the copolymer, the 1 -butene derived units are about 2 to 40 wt % of the copolymer, and the 2-butene derived units are about 2 to 20 wt % of the copolymer. Another suitable polybutene is a homopolymer or copolymer of isobutylene and 1-butene, wherein the isobutylene derived units are about 65 to 100 wt % of the homopolymer or copolymer, and the 1 -butene derived units are about 0 to 35 wt % of the copolymer. Commercial examples of a suitable processing oil includes the PARAPOL™ Series of processing oils or polybutene grades or Indopol™ from Soltex Synthetic Oils and Lubricants from BP/Innovene. The processing oil or oils can be present at about 1 to 60 phr, including about 2 to 40 phr, about 4 to 35 phr and about 5 to 30 phr in yet another embodiment.

## Cross-linking Agents/Co-agents

The elastomeric propylene-based polymer composition can optionally include one or more cross-linking agents, also referred to as co-agents. Suitable co-agents can include liquid and metallic multifunctional acrylates and methacrylates, functionalized polybutadiene resins, functionalized cyanurate, and allyl isocyanurate. More particularly, suitable coagents can include, but are not limited to polyfunctional vinyl or allyl compounds such as, for example, triallyl cyanurate, triallyl isocyanurate, pentaerthritol tetramethacrylate, ethylene glycol dimethacrylate, diallyl maleate, dipropargyl maleate, dipropargyl monoallyl cyanurate, azobisisobutyronitrile and the like, and combinations thereof. Commercially available cross-linking agents/co-agents can be purchased from Sartomer.

The elastomeric propylene-based polymer composition may contain about 0.1 wt % or greater of co-agent based on the total weight of polymer composition. The amount of co-agent(s) can be about 0.1 wt% to about 15 wt %, based on the total weight of polymer composition. In one or more embodiments, the amount of co-agent(s) can have a low of about 0.1 wt%, about 1.5 wt% or about 3.0 wt% to a high of about 4.0 wt %, about 7.0 wt %, or about 15 wt %, based on the total weight of blend. In one or more embodiments, the amount of co-agent (s) can have a low of about 2.0 wt %, about 3.0 wt % or about 5.0 wt % to a high of about 7.0 wt %, about 9.5 wt %, or about 12.5 wt %, based on the total weight of the polymer composition.

## Antioxidants

The elastomeric propylene-based polymer composition can optionally include one or more anti-oxidants. Suitable anti-oxidants can include hindered phenols, phosphites, hindered amines, Irgafos 168, Irganox 1010, Irganox 3790, Irganox B225, Irganox 1035, Irgafos 126, Irgastab 410, Chimassorb 944, etc. made by Ciba Geigy Corp. These may be added to the elastomeric composition to protect against degradation during shaping or fabrication operation and/or to better control the extent of chain degradation which can be especially useful where the elastomeric propylene-based polymer composition is exposed to e-beam.

The elastomeric propylene-based composition can contain at least about 0.1 wt% of antioxidant, based on the total weight of blend. In one or more embodiments, the amount of antioxidant(s) can be about 0.1 wt % to about 5 wt %, based on the total weight of blend. In one or more embodiments, the amount of antioxidant(s) can have a low of about 0.1 wt %, about 0.2 wt % or about 0.3 wt % to a high of about 1 wt %, about 2.5 wt %, or about 5 wt %, based on the total weight of blend. In one or more embodiments, the amount of antioxidant(s) is about 0.1 wt %, based on the total weight of blend. In one or more embodiments, the amount of antioxidant(s) is about 0.2 wt %, based on the total weight of blend. In one or more embodiments, the amount of antioxidant(s) is about 0.3 wt %, based on the total weight of blend. In one or more embodiments, the amount of antioxidant(s) is about 0.4 wt %, based on the total weight of blend. In one or more embodiments, the amount of antioxidant(s) is about 0.5 wt %, based on the total weight of blend.

**Blending and Additives**

In one or more embodiments, the individual materials and components, such as the propylene-based polymer and optionally the one or more polyolefinic thermoplastic resins, additional elastomeric component, additive oil, coagents, and anti-oxidants can be blended by melt-mixing to form a blend. Examples of machinery capable of generating the shear and mixing include extruders with kneaders or mixing elements with one or more mixing tips or flights, extruders with one or more screws, extruders of co or counter rotating type, Banbury mixer, Farrell Continuous mixer, and the Buss Kneader. The type and intensity of mixing, temperature, and residence time required can be achieved by the choice of one of the above machines in combination with the selection of kneading or mixing elements, screw design, and screw speed (<3000 RPM).

In one or more embodiments, the blend can include the propylene-based polymer in an amount having a low of about 60, about 70 or about 75 wt % to a high of about 80, about 90, or about 95 wt %. In one or more embodiments, the blend can include the one or more polyolefinic thermoplastic components in an amount having a low of about 5, about10 or about 20 wt % to a high of about 25, about 30, or about 75 wt%. In one or more embodiments, the blend can include the additional elastomeric component in an amount having from a low of about 5, about 10 or about 15 wt % to a high of about 20, about 35, or about 50 wt %.

In one or more embodiments, the co-agents, antioxidants, and/or other additives can be introduced at the same time as the other polymer components or later downstream in case of using an extruder or Buss kneader or only later in time. In addition to the co-agents and antioxidants described, other additives can include antiblocking agents, antistatic agents, ultraviolet stabilizers, foaming agents, and processing aids. The additives can be added to the blend in pure form or in master batches.

**Cured Products**

The formed article (e.g., extruded article) can be a fiber, yarn or film, and may be at least partially crosslinked or cured. Cross-linking provides the articles with heat resistance which is useful when the article, such as a fiber or yarn, will be exposed to higher temperatures. As used herein, the term "heat-resistant" refers to the ability of a polymer composition or an article formed from a polymer composition to pass the high temperature heat-setting and dyeing tests described herein.

As used herein, the terms "cured," "crosslinked," "at least partially cured," and "at least partially crosslinked" refer to a composition having at least about 2 wt % insolubles based on the total weight of the composition. The elastomeric polypropylene-based compositions described herein can be cured to a degree so as to provide at least about 3 wt %, or at least about 5 wt %, or at least about 10 wt %, or at least about 20 wt %, or at least about 35 wt %, or at least about 45 wt %, or at least about 65 wt %, or at least about 75 wt %, or at least about 85 wt %, or less than about 95 wt % insolubles using Xylene as the solvent by Soxhlet extraction.

In a particular embodiment, the crosslinking is accomplished by electron beam or simply "ebeam" after shaping or extruding the article. Suitable ebeam equipment is available from E-BEAM Services, Inc. In a particular embodiment, electrons are employed at a dosage of about 100 kGy or less in multiple exposures. The source can be any electron beam generator operating in a range of about 150 Kev to about 12 mega-electron volts (MeV) with a power output capable of supplying the desired dosage. The electron voltage can be adjusted to appropriate levels which may be, for example, about 100,000; about 300,000; about 1,000,000; about 2,000,000; about 3,000,000; about 6,000,000. A wide range of apparatus for irradiating polymers and polymeric articles is available.

Effective irradiation is generally carried out at a dosage between about 10 kGy (Kilogray) to about 350 kGy, preferably from about 20 to about 350 kGy, or from about 30 to about 250 kGy, or from about 40 to about 200 kGy. In a particular aspect of this embodiment, the irradiation is carried out at room temperature.

In another embodiment, crosslinking can be accomplished by exposure to one or more chemical agents in addition to the e-beam cure. Illustrative chemical agents include, but are not limited to, peroxides and other free radical generating agents, sulfur compounds, phenolic resins, and silicon hydrides. In a particular aspect of this embodiment, the crosslinking agent is either a fluid or is converted to a fluid such that it can be applied uniformly to the article. Fluid crosslinking agents include those compounds which are gases (e.g., sulfur dichloride), liquids (e.g., Trigonox C, available from Akzo Nobel), solutions (e.g., dicumyl peroxide in acetone, or suspensions thereof (e.g., a suspension or emulsion of dicumyl peroxide in water, or redox systems based on peroxides).

Illustrative peroxides include, but are not limited to, dicumyl peroxide, di-tert-butyl peroxide, t-butyl perbenzoate, benzoyl peroxide, cumene hydroperoxide, t-butyl peroctoate, methyl ethyl ketone peroxide, 2,5-dimethyl-2,5-di(tbutyl peroxy)hexane, lauryl peroxide, tert-butyl peracetate. When used, peroxide curatives are generally selected from organic peroxides. Examples of organic peroxides include, but are not limited to, di-tert-butyl peroxide, dicumyl peroxide, t-butylcumyl peroxide, $\alpha,\alpha$-bis(tert-butylperoxy) diisopropyl benzene, 2,5 dimethyl 2,5-di(t-butylperoxy)hexane, 1,1-di(t-butylperoxy)-3,3,5-trimethyl cyclohexane, -butyl-4, 4-bis(tert-butylperoxy) valerate, benzoyl peroxide, lauroyl peroxide, dilauroyl peroxide, 2,5-dimethyl-2,5-di(tert-butylperoxy) hexene-3, and mixtures thereof. Also, diaryl peroxides, ketone peroxides, peroxydicarbonates, peroxyesters, dialkyl peroxides, hydroperoxides, peroxyketals and mixtures thereof may be used.

In one or more embodiments, the crosslinking can be carried out using hydrosilylation techniques.

In one or more embodiments, the crosslinking can be carried out under an inert or oxygen-limited atmosphere. Suitable atmospheres can be provided by the use of helium, argon, nitrogen, carbon dioxide, xenon and/or a vacuum.

Crosslinking either by chemical agents or by irradiation can be promoted with a crosslinking catalyst, such as organic bases, carboxylic acids, and organometallic compounds including organic titanates and complexes or carboxylates of lead, cobalt, iron, nickel, zinc, and tin (such as dibutyltindilaurate, dioctyltinmaleate, dibutyltindiacetate, dibutyltindioctoate, stannous acetate, stannous octoate, lead naphthenate, zinc caprylate, cobalt naphthenate, and the like).

## Stretch Articles

Embodiments of the present disclosure include stretch articles including a substrate made, at least in part, from a substrate material and at least one stretch yarn including an elastomeric propylene-based polymer, as described above. In the stretch articles of the present disclosure, the stretch yarn can be bonded to the substrate and is capable of being mechanically bonded directly to the substrate surface. In embodiments, the mechanical bonding of the stretch yarn to the substrate material can be done without the use of adhesive. In some embodiments a substantially small amount of adhesive may be used to help maintain the location of the stretch yarn relative to the substrate material. In embodiments, at least a portion of the stretch yarn is mechanically bonded directly to the substrate surface and is substantially free of an adhesive.

In discussed above, the stretch yarn used in preparing the stretch articles of the present disclosure may have a variety of cross sectional shapes including, but not limited to, round, flat, oblong, or "tape-like." In an embodiment, the stretch yarn may have a traditional rounded/spherical cross section or variations of such a cross-section. In other embodiments, the stretch yarn has a flat or ribbon-like shape, such that the cross-sectional shape is elongated and/or oblong, with a rectangle or oval cross-section or variations thereof. In embodiments, the cross-sectional shape of the stretch yarn is elongated, and has a width dimension that is greater than a height dimension.

As described above, in some exemplary embodiments the elastomeric ethylene-propylene based polymer can be crosslinked. Thus, as described in greater detail above, in some embodiments the ethylene-propylene based polymer also includes a diene and/or a cross-linking agent. In embodiments, the diene is selected from the following: 5-ethylidene-2-norbornene (ENB); 1,4-hexadiene; 5-methylene-2-norbornene (MNB); 1,6-octadiene; 5-methyl-1,4-hexadiene; 3,7-dimethyl-1,6-octadiene; 1,3-cyclopentadiene; 1,4-cyclohexadiene; vinyl norbornene (VNB); dicyclopendadiene (DCPD), and combinations thereof. In embodiments, the cross-linking agent is selected from the following: multifunctional acrylates, multifunctional methacrylates, functionalized polybutadiene resins, functionalized cyanurate, and allyl isocyanurate, and combinations thereof.

In embodiments where the elastomeric polymer is crosslinkable by the inclusion of a diene and/or crosslinking agent, the method of producing the stretch article of the present disclosure may further include crosslinking the fiber at some point before or after incorporation of the stretch yarn into the stretch article. During the fiber-making process, the fiber may be crosslinked after spinning but before being wound onto the package, or, the fiber may be crosslinked at some point down-stream of fiber formation, such as before being incorporated into the stretch article of the present disclosure, or after the fiber has been incorporated into an end-use article. Crosslinking may be accomplished by various methods known to those of skill in the art, including, but not limited to heat, chemical catalysis, e-beaming, and the like, such as described above. In an embodiment, cross-linking is achieved by exposing the fiber of the present disclosure to e-beam radiation. Other cross linking methods includes gamma radiation, heat, UV light, chemical catalysis, or combinations thereof.

In embodiments, a substantially small amount of an adhesive may be used to help keep the yarn from moving about in between layers of the substrate material; thus, in such embodiments the yarn is in contact a substantially small amount of adhesive.

In other embodiments, different methods are used to help keep the yarn from lateral movement between the layers of substrate material and thus adhesive is unnecessary. In such embodiments, the stretch yarn is substantially free of adhesive. In such embodiments, ultrasonic techniques may be employed to create ultrasonic weld points or tack points along the stretch yarn, and/or to ultrasonically bond the layers of substrate material to each other at points adjacent to the stretch yarn, creating a "track" or "tunnel" in which the yarn is more-or-less entrapped, thereby retraining the movement of the yarn.

In an exemplary embodiment, the elastomeric propylene-based polymer stretch yarn is crosslinked before the yarn is incorporated and bonded to the stretch article. The crosslinking imparts the elastomeric polymer with a higher melting-point and improved stability, which renders it more durable for processing steps such as hot melt/thermoplastic bonding or ultrasonic bonding steps. Thus, in embodiments where the crosslinked stretch yarn is used, adhesive may be eliminated, since higher temperature thermoplastic bonding can be used without the potential problem that the applied heat will break the yarn, as might be possible with the lower-melt elastomer.

Additionally, with the non-crosslinked elastomeric propylene-based polymer yarn, it is generally advisable to take care during ultrasonic bonding to reduce or remove tension from the yarn during the ultrasonic bonding process to avoid potential breakage of the yarn. However, typically during manufacture of stretch articles, such as disposable diapers, the stretch yarn is bonded to the substrate material while the stretch yarn is in a substantially extended state so that, after bonding, the substrate material will be gathered between the bonds of the stretch yarn to form a stretch article. Thus, in embodiments, tension can be reduced or removed from the stretch yarn during ultrasonic bonding, or other bonding methods, by placing a "finger" on either side of the bond point to reduce tension on the yarn in between the "fingers" during bonding. After bonding, the fingers can be removed and the tension returns to the yarn. In embodiments, if crosslinked elastomeric propylene-based polymer yarn is used, the "fingering" process can be eliminated since the increased strength of the crosslinked yarn allows it to be ultrasonically bonded while in a substantially extended state without breakage. In embodiments, the crosslinked stretch yarn can be used and thermoplastic bonding and/or ultrasonic bonding can be done along the length of the yarn, resulting in a strong mechanical bond. In some such embodiments, the stretch yarn can be substantially free of an adhesive.

In embodiments where a non-crosslinkable elastomeric propylene-based polymer yarn is used, thermoplastic and/or ultrasonic bonding may be done on each end of the yarn, and/or at bond intervals along the length of the yarn to mechanically bond the yarn directly to the substrate material. However, in embodiments, the yarn may still be able to move around relative to the substrate material in the area between the bonds. Thus, in some embodiments, a relatively small amount of adhesive may be employed in addition to thermoplastic and/or ultrasonic bonding to help to keep the yarn from moving/slipping in relation to the substrate material (e.g., creep). The relatively small amount of adhesive is much smaller than the amount typically used to bond a stretch yarn to a laminate when the adhesive is the only or primary bonding method employed to attach the stretch yarn to the stretch article.

In embodiments of the disclosure, the stretch articles of the present disclosure can be formed into articles of manufacture, such as, but not limited to, disposable absorbent garments such as infant diapers or training pants, adult incontinence products, and other such products are well-known in the art. Construction of such articles is known to those of skill in the art, but is described briefly here. Typically, the chassis of such garments includes at least two layers of substrate materials: a liquid-permeable body-contacting liner sheet (or "top sheet"), a liquid-impermeable backing sheet (or "back sheet") (collectively the "sheets"), and a moisture-absorbent core fiber (or "absorbent core") that usually is made of a non-woven mat of randomly arranged fiber and is generally disposed between the top sheet and the back sheet. These garments typically further include various elastic components and fastening systems to facilitate use of the garments, and they often comprise additional sheets.

The substrate materials used to form the stretch articles of the present disclosure (e.g., materials suitable for use as the first and second layers of substrate material to which the stretch yarn is bonded) include, but are not limited to, nonwoven materials, such as spunbond or meltblown thermoplastic polymers, such as polyolefins; bonded carded webs; film materials, such as polyolefin, ethylene vinyl acetate, ethyl methacrylate, and polyester films; foam materials, such as polyolefin foams; woven materials, such as woven polypropylene, polyethylene or polyester fabrics; and composites and laminates of the above nonwoven, film, foam, and woven materials. In an exemplary embodiment of the present disclosure, the first and second layer of substrate materials are non-integrally formed. That is, the first and second layers of substrate material represent separate elements that are not joined other than by thermal, adhesive or similar attaching techniques; the layers are not formed from an integral piece of material through a folding process. In another specific embodiment of the present disclosure, the first and second substrate material layers are integrally formed from a single, integral piece of material through a folding process.

The first layer and/or the second layer of the stretch article may be stretchable, either elastically or extensible. In particular embodiments, either or both the first layer and the second layer can provide a stretch elongation that is no less than about 20%, alternatively, no less than about 60%; and finally, alternatively, no less than about 100%. In particular embodiments, the first layer or the second layer can provide a stretch elongation measured at 250 g/in that are no more than about 120%; alternatively, no more than about 100%; and finally, alternatively, no more than about 50%. Thus, the first layer or the second layer typically can provide a stretch elongation measured at 250 g/in that is no less than about 20% and no more than about 120%; although the approximate percent may vary according to the general design and intended use of the stretch article. Further, the stretch elongation of the first layer may be different than the stretch elongation of the second layer.

In a specific embodiment, substrate materials are formed from a nonwoven material such as a spunbond or meltblown polyethylene or polypropylene material. When it is desired to provide an elastic composite which is generally liquid pervious, the spunbond material may suitably be treated with a surfactant to render it generally hydrophilic. If it desired to produce an elastic composite that is generally liquid impervious, the substrate material, or one of the first or second layers of substrate material, may include a liquid-impervious film, such as a polyolefin film, or the first or second layers of fusible material may include a spunbond-meltblown-spunbond or other suitably liquid-impervious nonwoven.

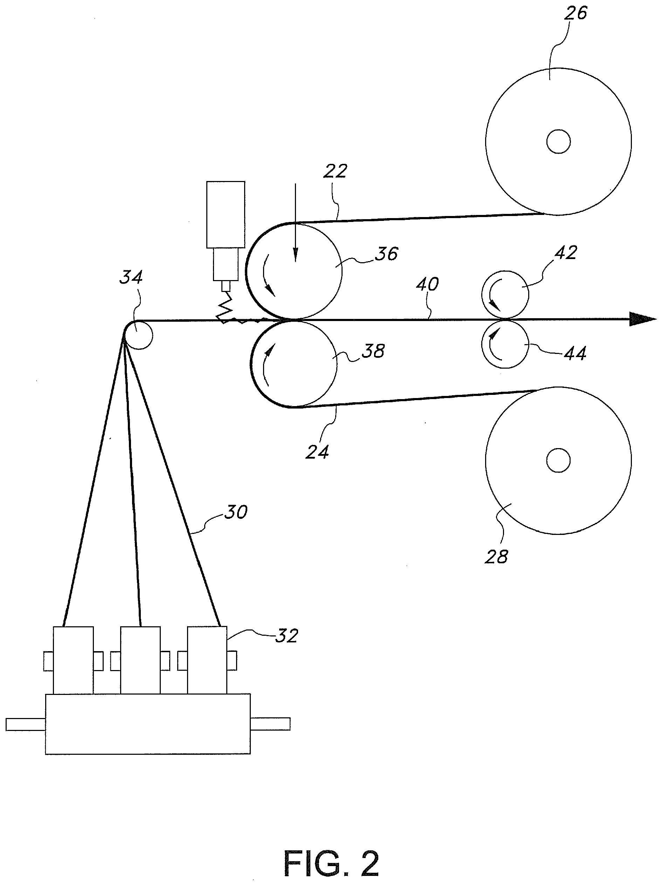

An embodiment of a method of making a stretch article of the present disclosure includes the following steps: providing a substrate material; providing a stretch yarn comprising an elastomeric propylene-based polymer; and bonding the stretch yarn to the substrate material such that at least a portion of the stretch yarn is mechanically bonded directly to the substrate material. FIG. 2 illustrates one embodiment of a method of bonding a stretch yarn between two layers of substrate material to form a stretch article.

As shown in FIG. 2, a stretch article or component (40) is made using the methods of the present disclosure where multiple strands of propylene-based elastomeric yarn (30) are sandwiched between two flat sheets of substrate materials (22 and 24) (e.g., nonwoven or extruded, etc.). Alternatively, one nonwoven sheet can be folded over to encase the strands of elastomeric yarn.

The combined yarn and substrate materials can be passed together in-between the surface of optionally heated nip rolls (36 and 38) to form the stretch article (40). Nip or roll surface pressure and temperature in the vicinity of the elastomeric yarns are controlled to either:1) cause the surface of the yarn strands to soften sufficiently to be modified or "molded" by the surface of the substrate materials, thereby increasing yarn-substrate bonding and reducing potential yarn creep, or 2) cause the surface of the yarn strands to slightly melt and bond directly to the substrate material, thereby increasing bond strength and reducing potential for yarn creep.

In an embodiment, a relatively small amount of adhesive (34) may be applied either directly on the yarn strands, on the surface of the substrate materials, or at the nip point of the rolls (36 and 38), just before the combined materials are passed together in between the surface of the rolls to form the stretch article (40).

In an embodiment, to maximize bonding, the bonded stretch-article (40) can be passed through another secondary set of optionally heated rolls (42 and 44) having controlled nip pressure and surface temperature to further increase mechanical bonding by the same mechanism.

Alternatively, the secondary set of heated rolls could be replaced by an ultrasonic welding apparatus (not shown), which can be controlled to ultrasonically weld the yarn strands to the substrate at regular intervals or desired spacing. In FIG. 2, yarn feeding with a positive feed unwinder is for illustration only. Over end take off methods may also be used to feed elastomeric yarn.

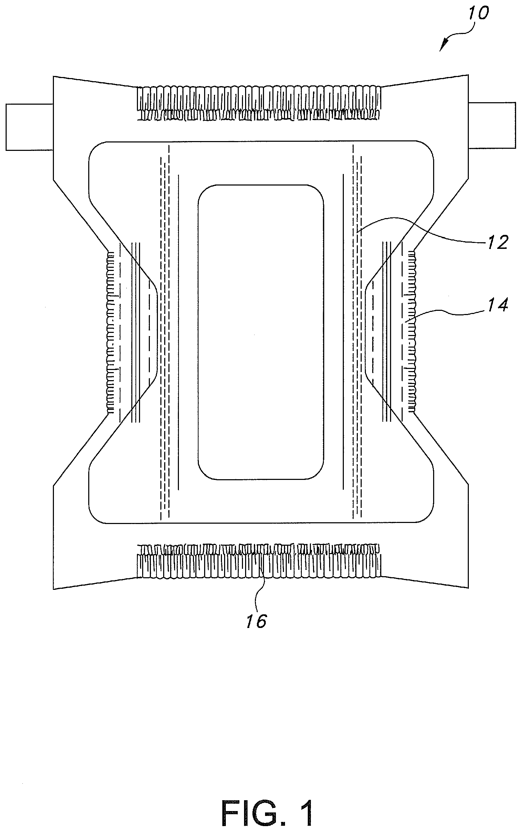

In another exemplary method of forming the stretch articles of the present disclosure mechanical bonding by heat and pressure is replaced by ultrasonic welding. In an exemplary method, one end of the sample can be run through an ultrasonic welder. The stretch yarn is then elongated (e.g., extended, stretched) and the other end is passed through the ultrasonic welder. A small amount of adhesive can be optionally added between the sandwiched substrate layers on or in the vicinity of the stretch yarn to further bond the sandwiched material together in a similar manner as described above. FIG. 1 illustrates the construction of a stretch article within a diaper (10). As shown, the stretch article of the present disclosure may be incorporated in the area commonly called an inner leg cuff/standing cuff (12) and/or in the area referred to as the outer leg cuff (14). The same method can also be used to attach waist or belly bands (16).

EXAMPLES

In the following examples, multiple elastomeric propylene-based polymer yarns were sandwiched between layers of a light-weight nonwoven fabric. The elastomeric stretch yarn was bonded to the layers of substrate material using the methods described below. The samples simulate the construction of a structure within a diaper commonly referred to as an inner leg cuff/standing cuff or outer leg cuff, described above and illustrated as inner leg cuff (12) and outer leg cuff (14) of the diaper (10) illustrated in FIG. 1.

**Example 1**

In this example, the process illustrated in Fig. 2 was used to construct a stretch article to simulate the construction of a structure within a diaper, such as a leg cuff or belly band. This example is described with reference to the system (20) illustrated in FIG. 2. First, two flat sheets of substrate material (first layer 22 and second layer 24) were supplied from large rolls (26 and 28, respectively). Multiple elastomeric propylene-based polymer yarns (30) were supplied from packages (32). The stretch yarns were passed between the first and second layers of substrate material such that the yarn was "sandwiched" between the two layers of substrate material. A small amount of adhesive (34) was applied to the yarn strands before the yarn and substrate materials are combined and passed together in between the surface of the heated nip rolls (36 and 38). The temperature and pressure were such to cause the surface of the yarn to melt and bond directly to the substrate material and/or to cause the surface of the yarn strands to soften sufficiently to be modified or molded by the surface of the substrate materials to increase yarn-substrate bonding. In order to increase bonding, the stretch article (40) is further passed through a secondary set of heated rolls (42 and 44) at controlled nip pressure and surface temperature to further increase the mechanical bonding between the stretch yarn and the substrate material. Alternatively (not shown), the second set of heated nip rolls can be replaced by an ultrasonic welding apparatus to ultrasonically weld the yarn strands to the substrate.

**Example 2**

In this example, the heat and pressure bonding is replaced by ultrasonic welding. In this example, at one end of the stretch yarns, the sample (with the yarns sandwiched between the two layers of substrate material) was run through an ultrasonic welder to bond the stretch yarns to the substrate material. The stretch yarns were then elongated (e.g., stretched), and the other end was passed through the ultrasonic welder, such that the stretch yarn was mechanically bonded to the substrate materials by ultrasonic weld points at each end of the stretch yarn. Then, the layers of substrate material were bonded together with either a small amount of adhesive or by ultrasonic weld points to entrap the bonded stretch yarn within the article.

It should be noted that ratios, concentrations, amounts, and other numerical data may be expressed herein in a range format. It is to be understood that such a range format is used for convenience and brevity, and thus, should be interpreted in a flexible manner to include not only the numerical values explicitly recited as the limits of the range, but also to include all the individual numerical values or sub-ranges encompassed within that range as if each numerical value and sub-range is explicitly recited. To illustrate, a concentration range of "about 0.1% to about 5%" should be interpreted to include not only the explicitly recited concentration of about 0.1 wt% to about 5 wt%, but also the individual concentrations (e.g., 1%, 2%, 3%, and 4%) and the sub-ranges (e.g., 0.5%, 1.1%, 2.2%, 3.3%, and 4.4%) within the indicated range. The term "about" can include $\pm 1\%$, $\pm 2\%$, $\pm 3\%$, $\pm 4\%$, $\pm 5\%$, $\pm 8\%$, or $\pm 10\%$, of the numerical value(s) being modified. In addition, the phrase "about 'x' to 'y'" includes "about 'x' to about 'y'".

**Claims**

1. A stretch article (40) comprising:

   a substrate comprising a substrate material (22, 24), and
   wherein the stretch article is **characterised in that** it comprises at least one stretch yarn (30) comprising more than one elastomeric fiber comprising an elastomeric propylene-based polymer, wherein the stretch yarn is bonded to the substrate and wherein the stretch yarn is capable of being mechanically bonded directly to the substrate surface.

2. The stretch article (40) of claim 1, further comprising a substantially small amount of an adhesive (34) in contact with the stretch yarn (30) and the substrate material (22, 24).

3. The stretch article (40) of claim 1, wherein the stretch yarn (30) is substantially free of an adhesive.

4. The stretch article (40) of claim 1, wherein the substrate comprises at least two layers of substrate material (22, 24) and wherein the stretch yarn (30) is positioned between two layers of substrate material (22, 24) and is bonded to each of the two layers.

5. The stretch article (40) of claim 1, wherein the substrate material (22, 24) comprises a non-woven or extruded polypropylene based fabric.

6. The stretch article (40) of claim 1, wherein the elastomeric propylene-based polymer further comprises a diene and a crosslinking agent and wherein the elastomeric propylene-based polymer is capable of being crosslinked.

7. The stretch article (40) of claim 6, wherein the yarn is at least partially crosslinked.

8. The stretch article (40) of claim 6,
   wherein the diene is selected from the group consisting of: 5-ethylidene-2-norbornene (ENB); 1,4-hexadiene; 5-methylene-2-norbornene (MNB); 1,6-octadiene; 5-methyl-1,4-hexadiene; 3,7-dimethyl-1,6-octadiene; 1,3-cyclopentadiene; 1,4-cyclohexadiene; vinyl norbornene (VNB); dicyclopendadiene (DCPD), and a combination thereof, and wherein the crosslinking agent is selected from the group consisting of: multifunctional acrylate, multifunctional methacrylate, functionalized polybutadiene resin, functionalized cyanurate, allyl isocyanurate, and a combination thereof.

9. The stretch article (40) of claim 1, wherein the stretch article (40) is a wearable disposable article selected from the group consisting of: a diaper, an incontinence article, and a personal hygiene article, wherein in one embodiment the stretch yarn forms part of a leg cuff portion, a waist band portion, or both, of the wearable disposable article.

**10.** A method of making a stretch article (40) comprising:

providing a substrate material (22, 24);
and wherein the method is **characterised by** the steps:

providing a stretch yarn (30) comprising more than one elastomeric fiber comprising an elastomeric propylene-based polymer; and
bonding the stretch yarn (30) to the substrate material (22, 24) such that at least a portion of the stretch yarn (30) is mechanically bonded directly to the substrate material (22, 24).

**11.** The method of claim 10, wherein an adhesive is not used to bond the stretch yarn (30) to the substrate material (22, 24).

**12.** The method of claim 10, wherein the stretch yarn (30) is substantially free of adhesive.

**13.** The method of claim 10, further comprising contacting one or more of the substrate material (22, 24) and the stretch yarn (30) with a substantially small amount of an adhesive (34) prior to bonding.

**14.** The method of claim 10, wherein the substrate material (22,24) comprises at least two layers of substrate material and wherein the stretch yarn (30) is positioned between two layers of substrate material (22, 24) and is bonded to each of the two layers.

**15.** The method of claim 10, wherein the bonding comprises mechanical bonding, or wherein in one embodiment the bonding comprises thermoplastic bonding, ultrasonic bonding, or a combination thereof.

**16.** The method of claim 10, further comprising passing the substrate material (22, 24) and stretch yarn (30) through an ultrasonic welding apparatus to ultrasonically weld at least a portion of the yarn to the substrate material (22, 24).

**17.** The method of claim 10, further comprising passing the substrate material (22, 24) and the stretch yarn (30) through heated nip rolls (36, 38) to at least partially bond the stretch yarn (30) to the substrate material (22, 24), wherein in one embodiment said method further comprises passing the substrate material (22, 24) and the stretch yarn (30) through a second set of heated nip rolls (42, 44) to further bond the stretch yarn (30) to the substrate material (22, 24), or wherein in a further embodiment said method further comprises passing the substrate material (22, 24) and the stretch yarn (30) through an ultrasonic welding apparatus to at least partially ultrasonically weld the yarn strands to the substrate.

**18.** The method of claim 10, wherein the elastomeric propylene-based polymer further comprises a diene and a crosslinking agent, wherein the elastomeric propylene-based polymer is capable of being crosslinked.

**19.** The method of claim 18, wherein the diene is selected from the group consisting of: 5-ethylidene-2-norbornene (ENB); 1,4-hexadiene; 5-methylene-2-norbornene (MNB); 1,6-octadiene; 5-methyl-1,4-hexadiene; 3,7-dimethyl-1,6-octadiene; 1,3-cyclopentadiene; 1,4-cyclohexadiene; vinyl norbornene (VNB); dicyclopendadiene (DCPD), and a combination thereof.

**20.** The method of claim 18, wherein the crosslinking agent is selected from the group consisting of: multifunctional acrylate, multifunctional methacrylate, functionalized polybutadiene resin, functionalized cyanurate, allyl isocyanurate, and a combination thereof.

**21.** The method of claim 18, further comprising subjecting the stretch article (40) to e-beam radiation to at least partially crosslink the stretch yarn (30).

**22.** The method of claim 18, further comprising at least partially crosslinking the stretch yarn (30), wherein crosslinking the yarn comprises subjecting the yarn to an energy that is selected from the group consisting of: an e-beaming energy, gamma radiation, an x ray energy, heat, UV light, and a combination thereof.

**23.** The method of claim 10, wherein the stretch article (40) is a wearable disposable article selected from the group consisting of: a diaper, an incontinence article, and a personal hygiene article, wherein in one embodiment the stretch yarn forms part of a leg cuff portion, a waist band portion, or both, of the wearable disposable article.

24. The method of claim 10, wherein the stretch yarn (30) comprises a stretch ribbon with a substantially elongated cross-sectional shape with a width and a height, wherein the width is greater than the height.

25. The stretch article (40) of claim 1 wherein at least a portion of the stretch yarn (30) is mechanically bonded directly to the substrate surface and is substantially free from an adhesive.

**Patentansprüche**

1. Stretchartikel (40), umfassend:

   ein Substrat, umfassend ein Substratmaterial (22, 24), und
   wobei der Stretchartikel **dadurch gekennzeichnet ist, dass** er wenigstens ein Stretchgarn (30) umfasst, das mehr als eine elastomere Faser umfasst, die ein elastomeres Polymer auf Propylenbasis umfasst, wobei das Stretchgarn mit dem Substrat verbunden ist und wobei das Stretchgarn imstande ist, mit der Substratoberfläche direkt mechanisch verbunden zu werden.

2. Stretchartikel (40) nach Anspruch 1, ferner umfassend eine im Wesentlichen kleine Menge eines Klebstoffs (34) in Kontakt mit dem Stretchgarn (30) und dem Substratmaterial (22, 24).

3. Stretchartikel (40) nach Anspruch 1, wobei das Stretchgarn (30) im Wesentlichen frei von einem Klebstoff ist.

4. Stretchartikel (40) nach Anspruch 1, wobei das Substrat wenigstens zwei Schichten des Substratmaterials (22, 24) umfasst und wobei das Stretchgarn (30) zwischen zwei Schichten des Substratmaterials (22, 24) angeordnet ist und mit jeder der zwei Schichten verbunden ist.

5. Stretchartikel (40) nach Anspruch 1, wobei das Substratmaterial (22, 24) ein Textilerzeugnis aus Faservlies oder auf Basis von extrudiertem Polypropylen umfasst.

6. Stretchartikel (40) nach Anspruch 1, wobei das elastomere Polymer auf Propylenbasis ferner ein Dien und ein Vernetzungsmittel umfasst und wobei das elastomere Polymer auf Propylenbasis imstande ist, vernetzt zu werden.

7. Stretchartikel (40) nach Anspruch 6, wobei das Garn wenigstens teilweise vernetzt ist.

8. Stretchartikel (40) nach Anspruch 6,
   wobei das Dien aus der Gruppe ausgewählt ist, die aus Folgendem besteht: 5-Ethyliden-2-norbornen (ENB); 1,4-Hexadien; 5-Methylen-2-norbornen (MNB); 1,6-Octadien; 5-Methyl-1,4-Hexadien; 3,7-Dimethyl-1,6-Octadien; 1,3-Cyclopentadien; 1,4-Cyclohexadien; Vinylnorbornen (VNB); Dicyclopendadien (DCPD) und einer Kombination davon, und
   wobei das Vernetzungsmittel aus der Gruppe ausgewählt ist, die aus Folgendem besteht: multifunktionalem Acrylat, multifunktionalem Methacrylat, funktionalisiertem Polybutadien-Harz, funktionalisiertem Cyanurat, Allylisocyanurat und einer Kombination davon.

9. Stretchartikel (40) nach Anspruch 1, wobei der Stretchartikel (40) ein tragbarer Einwegartikel ist, der aus der Gruppe ausgewählt ist, die aus Folgendem besteht: einer Windel, einem Inkontinenzartikel und einem Artikel für die persönliche Hygiene, wobei in einer Ausführungsform das Stretchgarn einen Teil eines Beinbündchenabschnitts, eines Taillenbundabschnitts oder beidem des tragbaren Einwegartikels bildet.

10. Verfahren zur Herstellung eines Stretchartikels (40), umfassend:

    Bereitstellen eines Substratmaterials (22, 24);
    und wobei das Verfahren durch die folgenden Schritte gekennzeichnet ist:

    Bereitstellen eines Stretchgarns (30), das mehr als eine elastomere Faser umfasst, die ein elastomeres Polymer auf Propylenbasis umfasst; und
    Verbinden des Stretchgarns (30) mit dem Substratmaterial (22, 24), so dass wenigstens ein Abschnitt des Stretchgarns (30) mit dem Substratmaterial (22, 24) direkt mechanisch verbunden ist.

11. Verfahren nach Anspruch 10, wobei kein Klebstoff verwendet wird, um das Stretchgarn (30) mit dem Substratmaterial (22, 24) zu verbinden.

12. Verfahren nach Anspruch 10, wobei das Stretchgarn (30) im Wesentlichen frei von Klebstoff ist.

13. Verfahren nach Anspruch 10, ferner umfassend das Inkontaktbringen von einem oder mehreren aus dem Substratmaterial (22, 24) und dem Stretchgarn (30) mit einer im Wesentlichen kleinen Menge eines Klebstoffs (34) vor dem Verbinden.

14. Verfahren nach Anspruch 10, wobei das Substratmaterial (22, 24) wenigstens zwei Schichten des Substratmaterials umfasst und wobei das Stretchgarn (30) zwischen zwei Schichten des Substratmaterials (22, 24) angeordnet wird und mit jeder der zwei Schichten verbunden wird.

15. Verfahren nach Anspruch 10, wobei das Verbinden mechanisches Verbinden umfasst oder wobei in einer Ausführungsform das Verbinden thermoplastisches Verbinden, Ultraschall-Verbinden oder eine Kombination davon umfasst.

16. Verfahren nach Anspruch 10, ferner umfassend das Durchlaufenlassen des Substratmaterials (22, 24) und des Stretchgarns (30) durch eine Ultraschallschweißvorrichtung, um wenigstens einen Abschnitt des Garns mit dem Substratmaterial (22, 24) mittels Ultraschall zu verschweißen.

17. Verfahren nach Anspruch 10, ferner umfassend das Durchlaufenlassen des Substratmaterials (22, 24) und des Stretchgarns (30) durch erhitzte Druckwalzen (36, 38), um das Stretchgarn (30) wenigstens teilweise mit dem Substratmaterial (22, 24) zu verbinden, wobei in einer Ausführungsform das Verfahren ferner das Durchlaufenlassen des Substratmaterials (22, 24) und des Stretchgarns (30) durch einen zweiten Satz von erhitzten Druckwalzen (42, 44) umfasst, um das Stretchgarn (30) weiter mit dem Substratmaterial (22, 24) zu verbinden, oder wobei in einer weiteren Ausführungsform das Verfahren ferner das Durchlaufenlassen des Substratmaterials (22, 24) und des Stretchgarns (30) durch eine Ultraschallschweißvorrichtung umfasst, um die Garnstränge wenigstens teilweise durch Ultraschall mit dem Substrat zu verschweißen.

18. Verfahren nach Anspruch 10, wobei das elastomere Polymer auf Propylenbasis ferner ein Dien und ein Vernetzungsmittel umfasst, wobei das elastomere Polymer auf Propylenbasis imstande ist, vernetzt zu werden.

19. Verfahren nach Anspruch 18, wobei das Dien aus der Gruppe ausgewählt ist, die aus Folgendem besteht: 5-Ethyliden-2-norbornen (ENB); 1,4-Hexadien; 5-Methylen-2-norbornen (MNB); 1,6-Octadien; 5-Methyl-1,4-Hexadien; 3,7-Dimethyl-1,6-Octadien; 1,3-Cyclopentadien; 1,4-Cyclohexadien; Vinylnorbornen (VNB); Dicyclopendadien (DCPD) und einer Kombination davon.

20. Verfahren nach Anspruch 18, wobei das Vernetzungsmittel aus der Gruppe ausgewählt ist, die aus Folgendem besteht: multifunktionalem Acrylat, multifunktionalem Methacrylat, funktionalisiertem Polybutadien-Harz, funktionalisiertem Cyanurat, Allylisocyanurat und einer Kombination davon.

21. Verfahren nach Anspruch 18, ferner umfassend das Unterziehen des Stretchartikels (40) einer Elektronenstrahlbestrahlung, um das Stretchgarn (30) wenigstens teilweise zu vernetzen.

22. Verfahren nach Anspruch 18, ferner umfassend das wenigstens teilweise Vernetzen des Stretchgarns (30), wobei das Vernetzen des Garns das Aussetzen des Garns gegenüber einer Energie umfasst, die aus der Gruppe ausgewählt ist, die aus Folgendem besteht: einer Elektronenstrahlenergie, einer Gammastrahlung, einer Röntgenstrahlenergie, Hitze, UV-Licht und einer Kombination davon.

23. Verfahren nach Anspruch 10, wobei der Stretchartikel (40) ein tragbarer Einwegartikel ist, der aus der Gruppe ausgewählt ist, die aus Folgendem besteht: einer Windel, einem Inkontinenzartikel und einem Artikel für die persönliche Hygiene, wobei in einer Ausführungsform das Stretchgarn einen Teil eines Beinbündchenabschnitts, eines Taillenbundabschnitts oder beidem des tragbaren Einwegartikels bildet.

24. Verfahren nach Anspruch 10, wobei das Stretchgarn (30) ein Stretchband mit einer im Wesentlichen langgestreckten Querschnittsform mit einer Breite und einer Höhe umfasst, wobei die Breite größer als die Höhe ist.

**25.** Stretchartikel (40) nach Anspruch 1, wobei wenigstens ein Abschnitt des Stretchgarns (30) mit der Substratoberfläche direkt mechanisch verbunden ist und frei von einem Klebstoff ist.


**Revendications**

**1.** Article extensible (40) comprenant :

un substrat comprenant un matériau de substrat (22, 24), et
l'article extensible étant **caractérisé en ce qu'**il comprend au moins un fil extensible (30) comprenant plus d'une fibre élastomère comprenant un polymère élastomère à base de propylène, le fil extensible étant fixé au substrat et le fil extensible étant capable d'être fixé mécaniquement directement à la surface du substrat.

**2.** Article extensible (40) selon la revendication 1, comprenant en outre une quantité sensiblement faible d'un adhésif (34) en contact avec le fil extensible (30) et le matériau de substrat (22, 24).

**3.** Article extensible (40) selon la revendication 1, dans lequel le fil extensible (30) est sensiblement exempt d'adhésif.

**4.** Article extensible (40) selon la revendication 1, dans lequel le substrat comprend au moins deux couches de matériau de substrat (22, 24) et dans lequel le fil extensible (30) est positionné entre deux couches de matériau de substrat (22, 24) et est fixé à chacune des deux couches.

**5.** Article extensible (40) selon la revendication 1, dans lequel le matériau de substrat (22, 24) comprend un tissu à base de polypropylène non tissé ou extrudé.

**6.** Article extensible (40) selon la revendication 1, dans lequel le polymère élastomère à base de propylène comprend en outre un diène et un agent de réticulation et dans lequel le polymère élastomère à base de propylène est capable d'être réticulé.

**7.** Article extensible (40) selon la revendication 6, dans lequel le fil est au moins en partie réticulé.

**8.** Article extensible (40) selon la revendication 6,
dans lequel le diène est sélectionné dans le groupe constitué du 5-éthylidène-2-norbornène (ENB), du 1,4-hexadiène, du 5-méthylène-2-norbornène (MNB), du 1,6-octadiène, du 5-méthyl-1,4-hexadiène, du 3,7-diméthyl-1,6-octadiène, du 1,3-cyclopentadiène, du 1,4-cyclohexadiène, du vinylnorbornène (VNB), du dicyclopendadiène (DCPD), et d'une combinaison de ceux-ci, et
dans lequel l'agent de réticulation est sélectionné dans le groupe constitué d'un acrylate multifonctionnel, d'un méthacrylate multifonctionnel, d'une résine de polybutadiène fonctionnalisée, d'un cyanurate fonctionnalisé, d'un isocyanurate d'allyle, et d'une combinaison de ceux-ci.

**9.** Article extensible (40) selon la revendication 1, l'article extensible (40) étant un article jetable pouvant être porté sélectionné dans le groupe constitué d'une couche, d'un article d'incontinence, et d'un article d'hygiène personnelle, dans une réalisation, le fil extensible faisant partie d'une partie enserrant la jambe, d'une bande enserrant la taille, ou des deux, de l'article jetable pouvant être porté.

**10.** Procédé de fabrication d'un article extensible (40) comprenant :

la fourniture d'un matériau de substrat (22, 24) ;
et le procédé étant **caractérisé par** les étapes suivantes :

fourniture d'un fil extensible (30) comprenant plus d'une fibre élastomère comprenant un polymère élastomère à base de propylène ; et
fixation du fil extensible (30) au matériau de substrat (22, 24) de telle sorte qu'au moins une partie du fil extensible (30) soit fixée mécaniquement directement au le matériau de substrat (22, 24).

**11.** Procédé selon la revendication 10, dans lequel on n'utilise pas d'adhésif pour fixer le fil extensible (30) au matériau de substrat (22, 24).

**12.** Procédé selon la revendication 10, dans lequel le fil extensible (30) est sensiblement exempt d'adhésif.

**13.** Procédé selon la revendication 10, comprenant en outre la mise en contact d'un ou plusieurs du matériau de substrat (22, 24) et du fil extensible (30) avec une quantité sensiblement faible d'adhésif (34) avant la fixation.

**14.** Procédé selon la revendication 10, dans lequel le matériau de substrat (22, 24) comprend au moins deux couches de matériau de substrat et dans lequel le fil extensible (30) est positionné entre deux couches de matériau de substrat (22, 24) et est fixé à chacune des deux couches.

**15.** Procédé selon la revendication 10, dans lequel la fixation comprend une fixation mécanique, ou dans lequel, dans une réalisation, la fixation comprend une fixation de type thermoplastique, une fixation de type ultrasonique, ou une combinaison de celles-ci.

**16.** Procédé selon la revendication 10, comprenant en outre le fait de faire passer le matériau de substrat (22, 24) et le fil extensible (30) dans un appareil de soudage ultrasonique pour souder par ultrasons au moins une partie du fil au matériau de substrat (22, 24).

**17.** Procédé selon la revendication 10, comprenant en outre le fait de faire passer le matériau de substrat (22, 24) et le fil extensible (30) entre des rouleaux pinceurs chauffants (36, 38) pour fixer au moins en partie le fil extensible (30) au matériau de substrat (22, 24), dans une réalisation, ledit procédé comprenant en outre le fait de faire passer le matériau de substrat (22, 24) et le fil extensible (30) entre une deuxième série de rouleaux pinceurs chauffants (42, 44) pour fixer davantage le fil extensible (30) au matériau de substrat (22, 24), ou bien, dans une autre réalisation, ledit procédé comprenant en outre le fait de faire passer le matériau de substrat (22, 24) et le fil extensible (30) dans un appareil de soudage ultrasonique pour souder au moins en partie par ultrasons les brins de fil au substrat.

**18.** Procédé selon la revendication 10, dans lequel le polymère élastomère à base de propylène comprend en outre un diène et un agent de réticulation, dans lequel le polymère élastomère à base de propylène est capable d'être réticulé.

**19.** Procédé selon la revendication 18, dans lequel le diène est sélectionné dans le groupe constitué du 5-éthylidène-2-norbornène (ENB), du 1,4-hexadiène, du 5-méthylène-2-norbornène (MNB), du 1,6-octadiène, du 5-méthyl-1,4-hexadiène, du 3,7-diméthyl-1,6-octadiène, du 1,3-cyclopentadiène, du 1,4-cyclohexadiène, du vinylnorbornène (VNB), du dicyclopendadiène (DCPD), et d'une combinaison de ceux-ci.

**20.** Procédé selon la revendication 18, dans lequel l'agent de réticulation est sélectionné dans le groupe constitué d'un acrylate multifonctionnel, d'un méthacrylate multifonctionnel, d'une résine de polybutadiène fonctionnalisée, d'un cyanurate fonctionnalisé, d'un isocyanurate d'allyle, et d'une combinaison de ceux-ci.

**21.** Procédé selon la revendication 18, comprenant en outre l'exposition de l'article extensible (40) à un rayonnement sous forme de faisceau d'électrons, pour réticuler au moins en partie le fil extensible (30).

**22.** Procédé selon la revendication 18, comprenant en outre la réticulation au moins en partie du fil extensible (30), dans lequel la réticulation du fil comprend l'exposition du fil à une énergie qui est sélectionnée dans le groupe constitué d'une énergie sous forme de faisceau d'électrons, d'un rayonnement gamma, d'une énergie sous forme de rayons x, de la chaleur, de la lumière UV, et d'une combinaison de ceux-ci.

**23.** Procédé selon la revendication 10, dans lequel l'article extensible (40) est un article jetable pouvant être porté sélectionné dans le groupe constitué d'une couche, d'un article d'incontinence, et d'un article d'hygiène personnelle, dans une réalisation, le fil extensible faisant partie d'une partie enserrant la jambe, d'une bande enserrant la taille, ou des deux, de l'article jetable pouvant être porté.

**24.** Procédé selon la revendication 10, dans lequel le fil extensible (30) comprend un ruban extensible ayant une forme de section transversale sensiblement allongée avec une largeur et une hauteur, la largeur étant supérieure à la hauteur.

**25.** Article extensible (40) selon la revendication 1, dans lequel au moins une partie du fil extensible (30) est fixée mécaniquement directement à la surface du substrat et est sensiblement exempte d'adhésif.

FIG. 1

FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4381781 A, Sciaraffa
- US 2007044905 A
- US 20040236042 A
- WO 05049672 A
- US 6881800 B
- US 20090298964 A
- WO 03040201 A
- US 6559262 B
- EP 1070087 A
- EP 1614699 A
- WO 0024792 A
- WO 0024793 A
- WO 0158912 A
- US 5153157 A
- US 5241025 A
- WO 9109882 A
- WO 9403506 A
- WO 9314132 A
- WO 9507941 A
- US 4540753 A
- US 6525157 B
- WO 0236651 A
- WO 03040202 A
- WO 03040095 A
- WO 03040233 A
- WO 03040442 A
- EP 1233191 A
- US 6770713 B
- US 2005215964 A
- EP 1017729 A
- US 6342565 B

### Non-patent literature cited in the description

- *Macromolecules,* 1988, vol. 21, 3360
- SYNTHETIC LUBRICANTS AND HIGH-PERFORMANCE FUNCTIONAL FLUIDS. Marcel Dekker, 1999, 357-392